# EUROPEAN PATENT SPECIFICATION

(11) **EP 3 802 798 B1**
(45) Date of publication and mention of the grant of the patent: **14.10.2026**
(21) Application number: 19811980.2
(22) Date of filing: 31.05.2019
(51) Int. Cl.: A61K 40/11, A61K 40/31, A61K 40/42, C12N 15/113, C07K 16/28, C07K 14/725, C07K 14/705, A61P 35/00

(54) **CHIMERIC ANTIGEN RECEPTOR T CELLS (CAR-T) FOR THE TREATMENT OF CANCER**
CHIMÄRE ANTIGEN-REZEPTOR-T-ZELLEN (CAR-T) ZUR BEHANDLUNG VON KREBS
LYMPHOCYTES T À RÉCEPTEURS D'ANTIGÈNES CHIMÉRIQUES (CAR-T) POUR LE TRAITEMENT DU CANCER

(30) Priority: 31.05.2018 US 201862678878 P; 31.01.2019 US 201962799513 P
(43) Date of publication of application: 14.04.2021
(60) Divisional application: 26203174.3
(73) Proprietor: Washington University, St. Louis, Missouri 63130 (US)
(72) Inventor: COOPER, Matthew, St. Louis, Missouri 63130 (US); DIPERSIO, John F., St. Louis, Missouri 63130 (US); O'NEAL, Julie, St. Louis, Missouri 63130 (US)
(74) Representative: Greaves Brewster LLP
(86) International application number: PCT/US2019/035010
(87) International publication number: WO 2019/232444

(56) References cited:
- WO-A1-2017/112877
- WO-A1-2018/027036
- WO-A1-2018/098306
- WO-A1-2019/232409
- WO-A1-2019/232425
- WO-A2-03/051926
- WO-A2-2019/232477
- US-A1- 2013 280 220
- COOPER MATTHEW L ET AL: "An "off-the-shelf" fratricide-resistant CAR-T for the treatment of T cell hematologic malignancies", LEUKEMIA, NATURE PUBLISHING GROUP UK, LONDON, vol. 32, no. 9, 20 February 2018 (2018-02-20), pages 1970 - 1983, XP036583020, ISSN: 0887-6924, [retrieved on 20180220], DOI: 10.1038/S41375-018-0065-5
- CARPENITO C ET AL: "Control of large, established tumor xenografts with genetically retargeted human T cells containing CD28 and CD137 domains", PROCEEDINGS OF THE NATIONAL ACADEMY OF SCIENCES, NATIONAL ACADEMY OF SCIENCES, vol. 106, no. 9, 3 March 2009 (2009-03-03), pages 3360 - 3365, XP002732434, ISSN: 0027-8424, [retrieved on 20090211], DOI: 10.1073/PNAS.0813101106
- GHOBADI ARMIN ET AL: "Phase 1/2 trial of anti-CD7 allogeneic WU-CART-007 for patients with relapsed/refractory T-cell malignancies", BLOOD ELSEVIER INC, UNITED STATES, vol. 146, no. 10, 2 June 2025 (2025-06-02), pages 1163 - 1173, ISSN: 0006-4971, [retrieved on 20250602], DOI: 10.1182/BLOOD.2025028387

## Description

Disclosed herein are genome-edited chimeric antigen receptor T cells (CAR-T) and methods of using them for immunotherapy. In particular, the disclosure relates to T cells that can be genetically modified to express one or more chimeric antigen receptors (CARs) and methods of using the same for the treatment of cancer.

T cells, a type of lymphocyte, play a central role in cell-mediated immunity. They are distinguished from other lymphocytes, such as B cells and natural killer cells (NK cells), by the presence of a T-cell receptor (TCR) on the cell surface. T helper cells (T_{H}), also called CD4⁺ T or CD4 T cells, express CD4 glycoprotein on their surface. Helper T cells are activated when exposed to peptide antigens presented by MHC (major histocompatibility complex) class II molecules. Once activated, these cells proliferate rapidly and secrete cytokines that regulate immune response. Cytotoxic T cells (T_{C}), also known as CD8⁺ T cells or CD8 T cells, express CD8 glycoprotein on the cell surface. The CD8⁺ T cells are activated when exposed to peptide antigens presented by MHC class I molecules. Memory T cells, a subset of T cells, persist long term and respond to their cognate antigen, thus providing the immune system with "memory" against past infections and/or tumor cells. Gamma delta (γδ) T cells are the prototype of 'unconventional' T cells and represent a relatively small subset of T cells in peripheral blood. They are defined by expression of heterodimeric T-cell receptors (TCRs) composed of y and δ chains. This sets them apart from the CD4⁺ helper T cells and CD8⁺ cytotoxic T cells. Viral-specific cytotoxic T lymphocytes are T cells with reactivity against viral antigens, notably Epstein-Barr virus (EBV) and cytomegalovirus (CMV).

The T cells described herein can be genetically modified to express chimeric antigen receptors (CARs), which are fusion proteins comprised of an antigen recognition moiety and T cell activation domains. T cells expressing CARs can recognize a specific protein, i.e., antigen on tumor cells. These T cells expressing CARs can be expanded in the laboratory prior to infusion into a patient.

Clinical trials have shown high response rates after anti-CD19 CAR infusion in patients with B cell malignancies, including diffuse large B cell lymphoma (DLBCL) and B cell-precursor acute lymphoblastic leukemia (ALL), resulting in two FDA approved therapies Yescarta^{™} (axicabtagene ciiloleucel, Kite Pharma/Gilead) and Kymriah^{™} (tisagenlecleucel, Novartis). Despite these successes, the development of CAR-T cell therapy against T cell malignancies has proven problematic, in part due to the shared expression of target antigens between malignant T cells and effector T cells. Among the most general challenges are: (1) the antigen target(s) for the chimeric antigen receptor(s); (2) CAR design, i.e., mono CAR, dual CAR, tandem CAR; and (3) tumor heterogeneity, particularly the variance in the surface expression of tumor antigens. Therefore, there remains a need for improved chimeric antigen receptor (CAR)-based immunotherapies, which utilize genome-editing and construction of mono, dual, and tandem CARs, for more effective, safe, and efficient targeting of cancers, including T-cell associated malignancies.

Cooper *et al.,* (2018) discloses an "off-the-shelf" fratricide-resistant CAR-T for the treatment of T cell hematologic malignancies. WO 03/051926 discloses an anti-CD7 fusion protein. Carpenito *et al.,* (2009) discloses control of large, established tumor xenografts with genetically retargeted human T cells containing CD28 and CD137 domains. WO 2019/232409 discloses methods for genome editing and activation of cells. WO 2019/232425 discloses methods for suppression of cytokine release syndrome in chimeric antigen receptor cell therapy. WO 2018/027036 discloses gene editing of CAR-T cells for the treatment of T cell malignancies with chimeric antigen receptors. US 2013/280220 discloses a chimeric antigen receptor for bispecific activation and targeting of T lymphocytes. WO 2017/112877 discloses chimeric antigen receptors and enhancement of anti-tumor activity. WO 2019/232477 discloses genomes-edited invariant natural killer T cells for the treatment of hematologic malignancies. WO 2018/098306 discloses blockade of CD7 expression and chimeric antigen receptors for immunotherapy of T-cell malignancies.

### SUMMARY OF THE INVENTION

The invention provides a chimeric antigen receptor (CAR)-comprising T cell (CAR-T cell), which comprises one or more chimeric antigen receptors (CARs) targeting one or more antigens expressed on a malignant T cell, wherein a CAR specifically binds to CD7 antigen and comprises the amino acid sequence 
MALPVTALLLPLALLLHAARPDIQMTQTTSSLSASLGDRVTISCSASQGISNYLNWYQQK
PDGTVKLLIYYTSSLHSGVPSRFSGSGSGTDYSLTISNLEPEDIATYYCQQYSKLPYTFGG
GTKLEIKRGGGGSGGGGSGGGGSGGGGSEVQLVESGGGLVKPGGSLKLSCAASGLTFSS
YAMSWVRQTPEKRLEWVASISSGGFTYYPDSVKGRFTISRDNARNILYLQMSSLRSEDT
 
AMYYCARDEVRGYLDVWGAGTTVTVSPRASTTTPAPRPPTPAPTIASQPLSLRPEACRP
AAGGAVHTRGLDFACDFWVLVVVGGVLACYSLLVTVAFIIFWVKRGRKKLLYIFKQPF
MRPVQTTQEEDGCSCRFPEEEEGGCELRVKFSRSADAPAYKQGQNQLYNELNLGRREE
YDVLDKRRGRDPEMGGKPRRKNPQEGLYNELQKDKMAEAYSEIGMKGERRRGKGHD
GLYQGLSTATKDTYDALHMQALPPR,
 and wherein the CAR-T cell is deficient in a subunit of the T cell receptor complex and is deficient in CD7.

The invention also provides a therapeutic composition comprising a population of CAR-T cells of the invention and at least one therapeutically acceptable carrier and/or adjuvant.

The invention also provides the CAR-T cell or the therapeutic composition of the invention for use in a method of treatment of cancer in a patient in need thereof.

### BRIEF DESCRIPTION OF THE DRAWINGS

**FIG. 1** shows a schematic of a dual CAR-T cell (dCAR-T cell).
**FIG. 2** shows a schematic of a tandem CAR-T cell (tCAR-T cell).

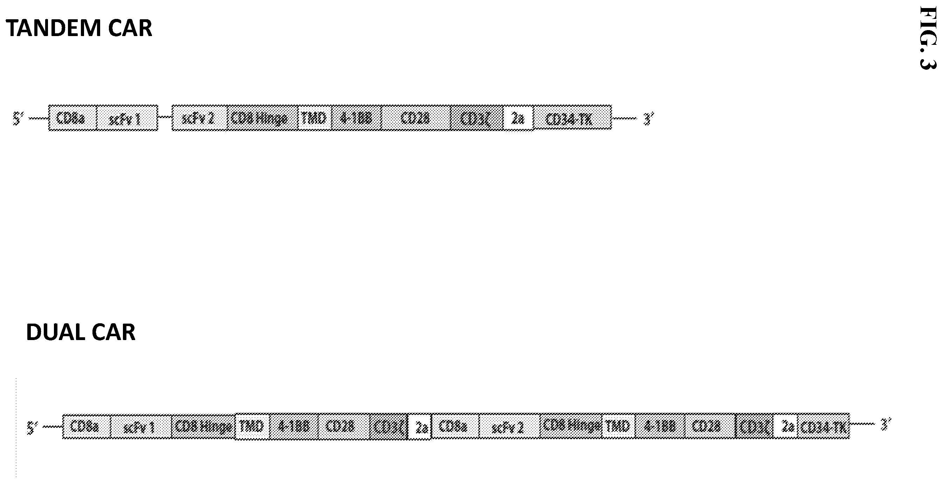

**FIG. 3** shows a schematic of dual and tandem CAR constructs.

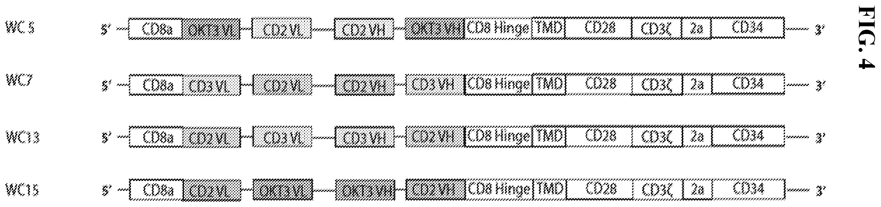

**FIG. 4** shows a schematic of tandem targeting CAR constructs.

### DETAILED DESCRIPTION

Disclosed herein is a genome-edited CAR-T cell, derived from a helper T cell, a cytotoxic T cell, a viral-specific cytotoxic T cell, a memory T cell, or a gamma delta (γδ) T cell, which comprise one or more chimeric antigen receptors (CARs) targeting one or more antigens, wherein the CAR-T cell is deficient in one or more antigens to which the one or more CARs specifically binds.

Also provided is a genome-edited CAR-T cell, derived from a helper T cell, a cytotoxic T cell, a viral-specific cytotoxic T cell, a memory T cell, or a gamma delta (γδ) T cell, which comprise one or more chimeric antigen receptors (CARs) targeting one or more antigens, wherein CAR-T cell is deficient in a subunit of the T cell receptor complex and one or more antigens to which the one or more CARs specifically binds.

Also provided is a CAR-T cell, derived from a helper T cell, a cytotoxic T cell, a viral-specific cytotoxic T cell, a memory T cell, or a gamma delta (γδ) T cell, in which the deficient subunit of the T cell receptor complex is selected from TCRα, TCRβ, TCRδ, TCRγ, CD3ε, CD3γ, CD3δ, and CD3ζ.

The chimeric antigen receptor specifically binds CD7.

In certain embodiments, CAR-T cell further comprises a suicide gene therapy system.

In certain embodiments, the endogenous T cell receptor-mediated signaling is blocked in the CAR-T cell.

In certain embodiments, the CAR-T cell does not induce alloreactivity or graft-versus-host disease.

In certain embodiments, the CAR-T cells do not induce fratricide.

Also provided is a dual CAR-T cell.

Also provided is a pharmaceutical composition comprising a population of CAR-T cells as disclosed herein, and at least one therapeutically acceptable carrier and/or adjuvant.

Also provided are methods for treating hematologic malignancies comprising administering a genome-edited CAR-T cell, a population of genome-edited CAR-T cells, wherein the population of genome-edited CAR-T cells are mono CAR-T cells, dual CAR-T cells, or tandem CAR-T cells as disclosed herein, or pharmaceutical compositions comprising them as disclosed herein to a patient in need thereof.

In certain embodiments, the hematologic malignancy is a T-cell malignancy.

In certain embodiments, the T cell malignancy is T-cell acute lymphoblastic leukemia (T-ALL).

In certain embodiments, the T cell malignancy is non-Hodgkin's lymphoma.

In certain embodiments, the T cell malignancy is T-cell chronic lymphocytic leukemia (T-CLL).

In certain embodiments, the hematologic malignancy is multiple myeloma.

In certain embodiments, the hematologic malignancy is acute myeloid leukemia (AML).

### CAR-T Cells

The present disclosure provides chimeric antigen receptor-bearing T cells (CAR-T cells), pharmaceutical compositions comprising them, and methods of immunotherapy for the treatment of cancer, specifically hematologic malignancies.

A CAR-T cell is a T cell which expresses a chimeric antigen receptor. The T cell expressing a CAR molecule may be a helper T cell, a cytotoxic T cell, a viral-specific cytotoxic T cell, a memory T cell, or a gamma delta (γδ) T cell.

A chimeric antigen receptor (CAR), is a recombinant fusion protein comprising: 1) an extracellular ligand-binding domain, i.e., an antigen-recognition domain, 2) a transmembrane domain, and 3) a signaling transducing domain.

The extracellular ligand-binding domain is an oligo- or polypeptide that is capable of binding a ligand. Preferably, the extracellular ligand-binding domain will be capable of interacting with a cell surface molecule which may be an antigen, a receptor, a peptide ligand, a protein ligand of the target, or a polypeptide of the target. The extracellular ligand-binding domain can specifically bind to an antigen with an affinity constant or affinity of interaction (K_{D}) between about 0.1 pM to about 10 pM, to about 0.1 pM to about 1 pM, or more preferably to about 0.1 pM to about 100 nM. Methods for determining the affinity constant or affinity of interaction (K_{D}) are well-known in the art. In some instances, the extracellular ligand-binding domain is chosen to recognize a ligand that acts as a cell surface marker on target cells associated with particular disease states.

The extracellular ligand-binding domain comprises a single chain antibody fragment (scFv) comprising the light (V_{L}) and the heavy (V_{H}) variable fragment joined by a linker and confers specificity for a T cell antigen. The chimeric antigen receptor of a CAR-T cell binds to an T cell-specific antigen expressed or overexpressed on a malignant T cell for which a CAR-T cell is deficient in the antigen (e.g., a genome-edited CAR-T cell).

Non-limiting examples of CAR-targeted antigens expressed on malignant T cells include CD5, CD7, CD2, CD4, and CD3.

The CAR-T cell of the claimed invention comprises a chimeric antigen receptor with an extracellular ligand-binding domain that specifically binds to CD7. In another words, the CAR which specifically binds CD7, comprises an extracellular ligand-binding domain comprising the polypeptide sequences with the amino acid sequence SEQ ID NO:20 and SEQ ID NO:21, and linked together by a flexible linker comprising the sequence (GGGGS)₄.

Non-limiting examples of CAR-targeted antigens expressed on the surface of leukemia cells (e.g., abnormal myeloblasts, red blood cells, or platelets) include CD123 (IL3RA), CD371 (CLL-1; CLEC12A), CD117 (c-kit), and CD135 (FLT3), CD7, and Tim3. A CAR may be constructed with an extracellular ligand-binding domain to target these antigens for treatment of leukemia, i.e., acute myeloid leukemia (AML).

Non-limiting examples of CAR-targeted antigens expressed on the surface of a multiple myeloma cell (e.g., a malignant plasma cell) include BCMA, CS1, CD38, CD79A, CD79B, CD138, and CD19. A CAR may be constructed with an extracellular ligand-binding domain to target these antigens for treatment of multiple myeloma. In another embodiment, the CAR may be constructed with a portion of the APRIL protein, targeting the ligand for the B-Cell Maturation Antigen (BCMA) and Transmembrane Activator and CAML Interactor (TACI), effectively co-targeting both BCMA and TACI for the treatment of multiple myeloma. A signal peptide directs the transport of a secreted or transmembrane protein to the cell membrane and/or cell surface to allow for correct localization of the polypeptide. Particularly, the signal peptide of the present disclosure directs the appended polypeptide, i.e., the CAR receptor, to the cell membrane wherein the extracellular ligand-binding domain of the appended polypeptide is displayed on the cell surface, the transmembrane domain of the appended polypeptide spans the cell membrane, and the signaling transducing domain of the appended polypeptide is in the cytoplasmic portion of the cell. The signal peptide according to the claimed invention is the signal peptide from human CD8α (SEQ ID NO: 1).

Typically, the extracellular ligand-binding domain is linked to the signaling transducing domain of the chimeric antigen receptor (CAR) by a transmembrane domain (Tm). The transmembrane domain traverses the cell membrane, anchors the CAR to the T cell surface, and connects the extracellular ligand-binding domain to the signaling transducing domain, impacting the expression of the CAR on the T cell surface.

The distinguishing feature of the transmembrane domain in the present disclosure is the ability to be expressed at the surface of an immune cell to direct an immune cell response against a pre-defined target cell. The transmembrane domain can be derived from natural or synthetic sources. Alternatively, the transmembrane domain of the present disclosure may be derived from any membrane-bound or transmembrane protein.

Non-limiting examples of transmembrane polypeptides of the present disclosure alpha, beta or zeta chain of the T-cell receptor, CD28, CD3 epsilon, CD45, CD4, *CDS,* CDS, CD9, CD16, CD22, CD33, CD37, CD64, CDS0, CD86, CD134, CD137 and CD154. Alternatively, the transmembrane domain can be synthetic and comprise predominantly hydrophobic amino acid residues (e.g., leucine and valine). The transmembrane domain according to the claimed invention is derived from CD28 (SEQ ID NO:3). The transmembrane domain can further comprise a hinge region between extracellular ligand-binding domain and said transmembrane domain. The term "hinge region" generally means any oligo- or polypeptide that functions to link the transmembrane domain to the extracellular ligand-binding domain. In particular, hinge region is used to provide more flexibility and accessibility for the extracellular ligand-binding domain. A hinge region may comprise up to 300 amino acids, preferably 10 to 100 amino acids and most preferably 25 to 50 amino acids. Hinge region may be derived from all or parts of naturally-occurring molecules such as CD28, 4-1BB (CD137), OX-40 (CD134), CD3ζ, the T cell receptor α or β chain, CD45, CD4, CD5, CD8, CD8α, CD9, CD16, CD22, CD33, CD37, CD64, CD80, CD86, ICOS, CD154 or from all or parts of an antibody constant region. Alternatively, the hinge region may be a synthetic sequence that corresponds to a naturally-occurring hinge sequence or the hinge region may be an entirely synthetic hinge sequence. The hinge domain according to the claimed invention comprises a part of human CD8α (SEQ ID NO 2).

A chimeric antigen receptor (CAR) of the present disclosure comprises a signal transducing domain or intracellular signaling domain of a CAR which is responsible for intracellular signaling following the binding of the extracellular ligand binding domain to the target resulting in the activation of the immune cell and immune response. In other words, the signal transducing domain is responsible for the activation of at least one of the normal effector functions of the immune cell in which the CAR is expressed. For example, the effector function of a T cell can be a cytolytic activity or helper T cell activity, including the secretion of cytokines. Thus, the term "signal transducing domain" refers to the portion of a protein which transduces the effector signal function signal and directs the cell to perform a specialized function.

Examples of signal transducing domains for use in a CAR can be the cytoplasmic sequences of the T cell receptor and co-receptors that act in concert to initiate signal transduction following antigen receptor engagement, as well as any derivate or variant of these sequences and any synthetic sequence that has the same functional capability. Signal transduction domain comprises two distinct classes of cytoplasmic signaling sequence, those that initiate antigen-dependent primary activation, and those that act in an antigen-independent manner to provide a secondary or co- stimulatory signal. Primary cytoplasmic signaling sequence can comprise signaling motifs which are known as immunoreceptor tyrosine-based activation motifs of ITAMs. ITAMs are well defined signaling motifs found in the intracytoplasmic tail of a variety of receptors that serve as binding sites for syk/zap70 class tyrosine kinases. Non-limiting examples of ITAM that can be used include those derived from TCRζ, FcRγ, FcRβ, FcRε, CD3γ, CD3δ, CD3ε, CDS, CD22, CD79a, CD79b and CD66d. The signaling transducing domain of the CAR according to the claimed invention comprises the CD3ζ signaling domain with the amino acid sequence with SEQ ID NO: 7.

In addition, the CAR-T cells of the present disclosure may further comprise one or more suicide gene therapy systems. Suitable suicide gene therapy systems known in the art include, but are not limited to, several herpes simplex virus thymidine kinase (HSVtk)/ganciclovir (GCV) or inducible caspase 9 proteins. In one embodiment, the suicide gene is a chimeric CD34/thymidine kinase.

T cells according to the claimed invention are deficient in an antigen to which the chimeric antigen receptor specifically binds and are therefore fratricide-resistant. In some embodiments, the antigen of the T cell is modified such that the chimeric antigen receptor no longer specifically binds the modified antigen. For example, the epitope of the antigen recognized by the chimeric antigen receptor may be modified by one or more amino acid changes (e.g., substitutions or deletions) or the epitope may be deleted from the antigen. In other embodiments, expression of the antigen is reduced in the T cell by at least 50%, at least 60%, at least 70%, at least 80%, at least 90% or more. Methods for decreasing the expression of a protein are known in the art and include, but are not limited to, modifying or replacing the promoter operably linked to the nucleic acid sequence encoding the protein. In still other embodiments, the T cell is modified such that the antigen is not expressed, e.g., by deletion or disruption of the gene encoding the antigen. In each of the above embodiments, the T cell may be deficient in one or preferably all the antigens to which the chimeric antigen receptor specifically binds. Methods for genetically modifying a T cell to be deficient in an antigen are well known in art, and non-limiting examples are provided above. In an exemplary embodiment, CRISPR/cas9 gene editing can be used to modify a T cell to be deficient in an antigen, for example as described below. Alternatively, TALENs may be used to edit genes.

In an variation of the method above, a construct encoding one or more protein expression blocker (PEBL) may be transduced into the cell, either as the editing step or part of the editing step, or as part of CAR transduction. Furthermore, PEBL and CAR expression can be combined in a single construct. Either of these methods may be achieved using the methods disclosed herein, and PEBLs may be produced for blockade of any of the targets of gene suppression disclosed herein.

The methods described above may be adapted to insert a CAR into a locus for a gene encoding an antigen, cell surface protein, or secretable protein, such as a cytokine. In this way, editing of the genome is effected by transfection of CAR. Thereafter, cells may be activated as described herein, removing separate genome editing step in certain embodiments. Ideally, such a step should be performed while cells are actively dividing. Such methods are also expected to result in robust expansion of engineered cells.

In certain circumstances, an T cell may be selected for deficiency in the antigen to which the chimeric antigen receptor specifically binds. Certain T cells will produce and display less of a given surface protein; instead if deleting or non-functionalizing the antigen that will be the target of the T-CAR, the T cell can be selected for deficiency in the antigen, and the population of antigen-deficient cells expanded for transduction of the CAR. Such a cell would also be fratricide-resistant.

**Table 1. Amino acid sequences of different CAR components.**

| Functional domains | SEQ ID NO: | Amino acid sequence |
|---|---|---|
| **CD8α signal peptide** | SEQ ID NO:1 | MALPVTALLLPLALLLHAARP |
| **CD8α hinge** | SEQ ID NO:2 | TTTPAPRPPTPAPTIASQPLSLRPEACRPAAGGA VHTRGLDFACD |
| **CD28 Transmembrane (Tₘ) domain** | SEQ ID NO:3 | FWVLVVVGGVLACYSLLVTVAFIIFWV |
| **Surface glycoprotein CD8 alpha chain isoform 1 precursor (NP_001139345.1)** | SEQ ID NO:4 | |
| **4-1BB costimulatory domain** | SEQ ID NO:5 | KRGRKKLLYIFKQPFMRPVQTTQEEDGCSCRFP EEEEGGCEL |
| **CD28 costimulatory domain** | SEQ ID NO:6 | RSKRSRLLHSDYMNMTPRRPGPTRKHYQPYAP PRDFAAYRS |
| **CD3 zeta (ζ)** | SEQ ID NO:7 | RVKFSRSADAPAYKQGQNQLYNELNLGRREEY DVLDKRRGRDPEMGGKPRRKNPQEGLYNELQ KDKMAEAYSEIGMKGERRRGKGHDGLYQGLS TATKDTYDALHMQALPPR |
| **P2A peptide** | SEQ ID NO:8 | GSGATNFSLLKQAGDVEENPGP |
| **(GGGGS)₄ linker** | SEQ ID NO:9 | GGGGSGGGGSGGGGSGGGGS |
| **hCD34** | SEQ ID NO:10 | MPRGWTALCLLSLLPSGFMSLDNNGTATPELP TQGTFSNVSTNVSYQETTTPSTLGSTSLHPVSQ |
| | | HGNEATTNITETTVKFTSTSVITSVYGNTNSSVQ SQTSVISTVFTTPANVSTPETTLKPSLSPGNVSD LSTTSTSLATSPTKPYTSSSPILSDIKAEIKCSGIR EVKLTQGICLEQNKTSSCAEFKKDRGEGLARV LCGEEQADADAGAQVCSLLLAQSEVRPQCLLL VLANRTEISSKLQLMKKHQSDLKKLGILDFTEQ DVASHQSYSQKTLIALVTSGALLAVLGITGYFL MNRRSWSPI |
| **Human-Herpes Simplex Virus-1 (HSV) - thymidine kinase (TK)** | SEQ ID NO:11 | MPRGWTALCLLSLLPSGFMSLDNNGTATPELP TQGTFSNVSTNVSYQETTTPSTLGSTSLHPVSQ HGNEATTNITETTVKFTSTSVITSVYGNTNSSVQ SQTSVISTVFTTPANVSTPETTLKPSLSPGNVSD LSTTSTSLATSPTKPYTSSSPILSDIKAEIKCSGIR EVKLTQGICLEQNKTSSCAEFKKDRGEGLARV LCGEEQADADAGAQVCSLLLAQSEVRPQCLLL VLANRTEISSKLQLMKKHQSDLKKLGILDFTEQ DVASHQSYSQKTLIALVTSGALLAVLGITGYFL MNRRSWSPTGEGGGGDLGGVKLPHLFGKRL VEARMASYPCHQHASAFDQAARSRGHSNRRT ALRPRRQQEATEVRLEQKMPTLLRVYIDGPHG MGKTTTTQLLVALGSRDDIVYVPEPMTYWQV LGASETIANIYTTQHRLDQGEISAGDAAVVMTS AQITMGMPYAVTDAVLAPHVGGEAGSSHAPPP ALTLLLDRHPIAVMLCYPAARYLMGSMTPQAV LAFVALIPPTLPGTNIVLGALPEDRHIDRLAKRQ RPGERLDLAMLAAIRRVYGLLANTVRYLQGGG SWWEDWGQLSGTAVPPQGAEPQSNAGPRPHIG DTLFTLFRAPELLAPNGDLYNVFAWALDVLAK RLRPMHVFILDYDQSPAGCRDALLQLTSGMVQ THVTTPGSIPTICDLARTFAREMGEAN |

**Table 2. Amino acid sequences of the variable heavy (V_{H}) and variable light (V_{L}) chains of the scFvs.**

| ScFv sequences | SEQ ID NO: | Amino acid sequence |
|---|---|---|
| **CD7 heavy chain variable region** | SEQ ID NO:20 | EVQLVESGGGLVKPGGSLKLSCAASGLTFSSY AMSWVRQTPEKRLEWVASISSGGFTYYPDSV KGRFTISRDNARNILYLQMSSLRSEDTAMYYC ARDEVRGYLDVWGAGTTVTVS |
| **CD7 light chain variable region** | SEQ ID NO:21 | DIQMTQTTSSLSASLGDRVTISCSASQGISNYL NWYQQKPDGTVKLLIYYTSSLHSGVPSRFSGS GSGTDYSLTISNLEPEDIATYYCQQYSKLPYTF GGGTKLEIKR |

### Mono CAR-T Cells (mCAR-T)

The CAR-T cells encompassed by the present disclosure are deficient in one or more antigens to which the chimeric antigen receptor specifically binds and are therefore fratricide-resistant. In some embodiments, the one or more antigens of the T cell is modified such the chimeric antigen receptor no longer specifically binds the one or more modified antigens. For example, the epitope of the one or more antigens recognized by the chimeric antigen receptor may be modified by one or more amino acid changes (e.g., substitutions or deletions) or the epitope may be deleted from the antigen. In other embodiments, expression of the one or more antigens is reduced in the T cell by at least 50%, at least 60%, at least 70%, at least 80%, at least 90% or more. Methods for decreasing the expression of a protein are known in the art and include, but are not limited to, modifying or replacing the promoter operably linked to the nucleic acid sequence encoding the protein. In still other embodiments, the T cell is modified such that the one or more antigens is not expressed, e.g., by deletion or disruption of the gene encoding the one or more antigens. In each of the above embodiments, the CAR-T cell may be deficient in one or preferably all the antigens to which the chimeric antigen receptor specifically binds. The methods to genetically modify a T cell to be deficient in one or more antigens are well known in art and non-limiting examples are provided herein. In embodiments described in Examples 1-6, the CRISPR-Cas9 system is used to modify a T cell to be deficient in one or more antigens.

CAR-T cells according to the claimed invention are further deficient in endogenous T cell receptor (TCR) signaling as a result of deleting a part of the T Cell Receptor (TCR)-CD3 complex. It is be desirable to eliminate or suppress endogenous TCR signaling in CAR-T cells disclosed herein. For example, decreasing or eliminating endogenous TCR signaling in CAR-T cells may prevent or reduce graft versus host disease (GvHD) when allogenic T cells are used to produce the CAR-T cells. Methods for eliminating or suppressing endogenous TCR signaling are known in the art and include, but are not limited to, deleting a part of the TCR-CD3 receptor complex, e.g., the TCR receptor alpha chain (TRAC), the TCR receptor beta chain (TCRβ), TCRδ, TCRγ, CD3ε, CD3γ, and/or CD3δ. Deleting a part of the TCR receptor complex may block TCR mediated signaling and may thus permit the safe use of allogeneic T cells as the source of CAR-T cells without inducing life-threatening GvHD.

In addition, the CAR-T cells encompassed by the present disclosure may further comprise one or more suicide genes as described herein.

The claimed invention provides a T cell comprising a chimeric antigen receptor that specifically binds CD7, wherein the T cell is deficient in CD7, e.g., CD7ΔCART7 cell. In non-limiting examples the deficiency in CD7 resulted from (a) modification of CD7 expressed by the T cell such that the chimeric antigen receptor no longer specifically binds the modified CD7, (b) modification of the T cell such that expression of the antigen is reduced in the T cell by at least 50%, at least 60%, at least 70%, at least 80%, at least 90% or more, or (c) modification of the T cell such that CD7 is not expressed (e.g., by deletion or disruption of the gene encoding CD7). In further embodiments, the T cell comprises a suicide gene and a modification such that endogenous T cell receptor (TCR) mediated signaling is blocked in the T cell. In non-limiting examples, a protein coding sequence of a modified Human-Herpes Simplex Virus-1-thymidine kinase (TK) gene fused in-frame to the extracellular and transmembrane domains of the human CD34 cDNA is expressed in CD7ΔCART7 cells.

Disclosed are CAR amino acid sequences that can be expressed on the surface of a genome-edited CAR-T cell derived from a cytotoxic T cell, a memory T cell, or a gamma delta (γδ) T cell.

**Table 3. Amino Acid Sequences of Mono Chimeric Antigen Receptors (CARs).**

| | | |
|---|---|---|
| Mono CAR Constructs | SEQ ID NO: | Amino acid sequence |
| **CD7-CAR-4-1BB_CD34** | SEQ ID NO:32 | MALPVTALLLPLALLLHAARPDIQMTQTTSSLSASLG DRVTISCSASQGISNYLNWYQQKPDGTVKLLIYYTSS LHSGVPSRFSGSGSGTDYSLTISNLEPEDIATYYCQQY SKLPYTFGGGTKLEIKRGGGGSGGGGSGGGGSGGGG SEVQLVESGGGLVKPGGSLKLSCAASGLTFSSYAMS WVRQTPEKRLEWVASISSGGFTYYPDSVKGRFTISRD NARNILYLQMSSLRSEDTAMYYCARDEVRGYLDVW GAGTTVTVSPRASTTTPAPRPPTPAPTIASQPLSLRPEA CRPAAGGAVHTRGLDFACDFWVLVVVGGVLACYSL LVTVAFIIFWVKRGRKKLLYIFKQPFMRPVQTTQEED GCSCRFPEEEEGGCELRVKFSRSADAPAYKQGQNQL YNELNLGRREEYDVLDKRRGRDPEMGGKPRRKNPQ EGLYNELQKDKMAEAYSEIGMKGERRRGKGHDGLY QGLSTATKDTYDALHMQALPPRRTDGSGATNFSLLK QAGDVEENPGPVSEAMPRGWTALCLLSLLPSGFMSL DNNGTATPELPTQGTFSNVSTNVSYQETTTPSTLGSTS LHPVSQHGNEATTNITETTVKFTSTSVITSVYGNTNSS VQSQTSVISTVFTTPANVSTPETTLKPSLSPGNVSDLS TTSTSLATSPTKPYTSSSPILSDIKAEIKCSGIREVKLTQ GICLEQNKTSSCAEFKKDRGEGLARVLCGEEQADAD AGAQVCSLLLAQSEVRPQCLLLVLANRTEISSKLQLM KKHQSDLKKLGILDFTEQDVASHQSYSQKTLIALVTS GALLAVLGITGYFLMNRRSWSPI |
| **CD7-CAR-4-1BB_CD34_TK** | SEQ ID NO:33 | MALPVTALLLPLALLLHAARPDIQMTQTTSSLSASLG DRVTISCSASQGISNYLNWYQQKPDGTVKLLIYYTSS LHSGVPSRFSGSGSGTDYSLTISNLEPEDIATYYCQQY SKLPYTFGGGTKLEIKRGGGGSGGGGSGGGGSGGGG SEVQLVESGGGLVKPGGSLKLSCAASGLTFSSYAMS |
| | | WVRQTPEKRLEWVASISSGGFTYYPDSVKGRFTISRD NARNILYLQMSSLRSEDTAMYYCARDEVRGYLDVW GAGTTVTVSPRASTTTPAPRPPTPAPTIASQPLSLRPEA CRPAAGGAVHTRGLDFACDFWVLVVVGGVLACYSL LVTVAFIIFWVKRGRKKLLYIFKQPFMRPVQTTQEED GCSCRFPEEEEGGCELRVKFSRSADAPAYKQGQNQL YNELNLGRREEYDVLDKRRGRDPEMGGKPRRKNPQ EGLYNELQKDKMAEAYSEIGMKGERRRGKGHDGLY QGLSTATKDTYDALHMQALPPRRTDGSGATNFSLLK QAGDVEENPGPVSEAMPRGWTALCLLSLLPSGFMSL DNNGTATPELPTQGTFSNVSTNVSYQETTTPSTLGSTS LHPVSQHGNEATTNITETTVKFTSTSVITSVYGNTNSS VQSQTSVISTVFTTPANVSTPETTLKPSLSPGNVSDLS TTSTSLATSPTKPYTSSSPILSDIKAEIKCSGIREVKLTQ GICLEQNKTSSCAEFKKDRGEGLARVLCGEEQADAD AGAQVCSLLLAQSEVRPQCLLLVLANRTEISSKLQLM KKHQSDLKKLGILDFTEQDVASHQSYSQKTLIALVTS GALLAVLGITGYFLMNRRSWSPTGEGGGGDLGGV KLPHLFGKRLVEARMASYPCHQHASAFDQAARSRG HSNRRTALRPRRQQEATEVRLEQKMPTLLRVYIDGP HGMGKTTTTQLLVALGSRDDIVYVPEPMTYWQVLG ASETIANIYTTQHRLDQGEISAGDAAVVMTSAQITMG MPYAVTDAVLAPHVGGEAGSSHAPPPALTLLLDRHPI AVMLCYPAARYLMGSMTPQAVLAFVALIPPTLPGTN IVLGALPEDRHIDRLAKRQRPGERLDLAMLAAIRRVY GLLANTVRYLQGGGSWWEDWGQLSGTAVPPQGAEP QSNAGPRPHIGDTLFTLFRAPELLAPNGDLYNVFAWA LDVLAKRLRPMHVFILDYDQSPAGCRDALLQLTSGM VQTHVTTPGSIPTICDLARTFAREMGEAN |
| **CD7-CAR-CD28_CD34** | SEQ ID NO:34 | MALPVTALLLPLALLLHAARPDIQMTQTTSSLSASLG DRVTISCSASQGISNYLNWYQQKPDGTVKLLIYYTSS LHSGVPSRFSGSGSGTDYSLTISNLEPEDIATYYCQQY SKLPYTFGGGTKLEIKRGGGGSGGGGSGGGGSGGGG SEVQLVESGGGLVKPGGSLKLSCAASGLTFSSYAMS WVRQTPEKRLEWVASISSGGFTYYPDSVKGRFTISRD NARNILYLQMSSLRSEDTAMYYCARDEVRGYLDVW GAGTTVTVSPRASTTTPAPRPPTPAPTIASQPLSLRPEA CRPAAGGAVHTRGLDFACDFWVLVVVGGVLACYSL LVTVAFIIFWVRSKRSRLLHSDYMNMTPRRPGPTRKH YQPYAPPRDFAAYRSRVKFSRSADAPAYKQGQNQLY NELNLGRREEYDVLDKRRGRDPEMGGKPRRKNPQE GLYNELQKDKMAEAYSEIGMKGERRRGKGHDGLYQ GLSTATKDTYDALHMQALPPRRTDGSGATNFSLLKQ AGDVEENPGPVSEAMPRGWTALCLLSLLPSGFMSLD NNGTATPELPTQGTFSNVSTNVSYQETTTPSTLGSTSL HPVSQHGNEATTNITETTVKFTSTSVITSVYGNTNSSV QSQTSVISTVFTTPANVSTPETTLKPSLSPGNVSDLSTT STSLATSPTKPYTSSSPILSDIKAEIKCSGIREVKLTQGI CLEQNKTSSCAEFKKDRGEGLARVLCGEEQADADAG AQVCSLLLAQSEVRPQCLLLVLANRTEISSKLQLMKK HQSDLKKLGILDFTEQDVASHQSYSQKTLIALVTSGA LLAVLGITGYFLMNRRSWSPI |
| **CD7-CAR-CD28_CD34_TK** | SEQ ID NO:35 | MALPVTALLLPLALLLHAARPDIQMTQTTSSLSASLG DRVTISCSASQGISNYLNWYQQKPDGTVKLLIYYTSS LHSGVPSRFSGSGSGTDYSLTISNLEPEDIATYYCQQY SKLPYTFGGGTKLEIKRGGGGSGGGGSGGGGSGGGG SEVQLVESGGGLVKPGGSLKLSCAASGLTFSSYAMS WVRQTPEKRLEWVASISSGGFTYYPDSVKGRFTISRD NARNILYLQMSSLRSEDTAMYYCARDEVRGYLDVW |
| | | GAGTTVTVSPRASTTTPAPRPPTPAPTIASQPLSLRPEA CRPAAGGAVHTRGLDFACDFWVLVVVGGVLACYSL LVTVAFIIFWVRSKRSRLLHSDYMNMTPRRPGPTRKH YQPYAPPRDFAAYRSRVKFSRSADAPAYKQGQNQLY NELNLGRREEYDVLDKRRGRDPEMGGKPRRKNPQE GLYNELQKDKMAEAYSEIGMKGERRRGKGHDGLYQ GLSTATKDTYDALHMQALPPRRTDGSGATNFSLLKQ AGDVEENPGPVSEAMPRGWTALCLLSLLPSGFMSLD NNGTATPELPTQGTFSNVSTNVSYQETTTPSTLGSTSL HPVSQHGNEATTNITETTVKFTSTSVITSVYGNTNSSV QSQTSVISTVFTTPANVSTPETTLKPSLSPGNVSDLSTT STSLATSPTKPYTSSSPILSDIKAEIKCSGIREVKLTQGI CLEQNKTSSCAEFKKDRGEGLARVLCGEEQADADAG AQVCSLLLAQSEVRPQCLLLVLANRTEISSKLQLMKK HQSDLKKLGILDFTEQDVASHQSYSQKTLIALVTSGA LLAVLGITGYFLMNRRSWSPTGEGGGGDLGGVKLP HLFGKRLVEARMASYPCHQHASAFDQAARSRGHSN RRTALRPRRQQEATEVRLEQKMPTLLRVYIDGPHGM GKTTTTQLLVALGSRDDIVYVPEPMTYWQVLGASET IANIYTTQHRLDQGEISAGDAAVVMTSAQITMGMPY AVTDAVLAPHVGGEAGSSHAPPPALTLLLDRHPIAV MLCYPAARYLMGSMTPQAVLAFVALIPPTLPGTNIVL GALPEDRHIDRLAKRQRPGERLDLAMLAAIRRVYGL LANTVRYLQGGGSWWEDWGQLSGTAVPPQGAEPQS NAGPRPHIGDTLFTLFRAPELLAPNGDLYNVFAWAL DVLAKRLRPMHVFILDYDQSPAGCRDALLQLTSGMV QTHVTTPGSIPTICDLARTFAREMGEAN |

In a similar manner, other mono-CAR-T cells may be constructed and are given below in Table 4.

**Table 4. Mono-CARs and CAR-Ts.**

| **Example** | **Antigen Target of CAR-T cells** | **Antigen Deletion/ Suppression** |
|---|---|---|
| **M52** | CD7 | CD7 |
| **M53** | CD7 | CD7 + TRAC |
| **M54** | CD7 | CD7 + CD3ε |

### Dual CAR-T Cells (dCAR-T)

A dual CAR-T cell (dCAR-T) may be generated by cloning a protein encoding sequence of a first extracellular ligand-binding domain into a lentiviral vector containing one or more costimulatory domains and a signaling transducing domain and cloning a second protein encoding sequence of a second extracellular ligand-binding domain into the same lentiviral vector containing an additional one or more costimulatory domains and a signaling transducing domain resulting in a plasmid from which the two CAR constructs are expressed from the same vector.

In a second embodiment, the disclosure provides an engineered T cell comprising a dCAR that specifically binds CD7 and TCR receptor alpha chain (TRAC), wherein the T cell is deficient in CD7 and TRAC, e.g., **CD7*TRAC**-dCARTACD7ATRAC cell. In non-limiting examples the deficiency in CD7 and the TCR receptor alpha chain (TRAC) resulted from (a) modification of CD7 and the TCR receptor alpha chain (TRAC) expressed by the T cell such that the chimeric antigen receptor no longer specifically binds the modified CD7 and the TCR receptor alpha chain (TRAC), (b) modification of the T cell such that expression of the CD7 and the TCR receptor alpha chain (TRAC) is reduced in the T cell by at least 50%, at least 60%, at least 70%, at least 80%, at least 90% or more, or (c) modification of the T cell such that CD7 and the TCR receptor alpha chain (TRAC) is not expressed (e.g., by deletion or disruption of the gene encoding CD7 and / or the TCR receptor alpha chain (TRAC). In further embodiments, the T cell comprises a suicide gene. In non-limiting examples, a protein coding sequence of a modified Human-Herpes Simplex Virus-1-thymidine kinase (TK) gene is fused in-frame to the extracellular and transmembrane domains of the human CD34 cDNA and is expressed in the CD7*TRAC-dCARTΔCD7ΔTRAC cells.

In another embodiment, the disclosure provides an engineered T cell comprising a dCAR that specifically binds CD7 and the TCR beta (β) chain, wherein the T cell is deficient in CD5 and TCR beta, e.g., **CD7*TCRβ**-dCARTΔCD7ΔTCRβ cell. In non-limiting examples the deficiency in CD7 and the TCRβ chain resulted from (a) modification of CD7 and TCRβ expressed by the T cell such that the chimeric antigen receptor no longer specifically binds the modified CD7 and TCRβ, (b) modification of the T cell such that expression of the CD7 and TCRβ is reduced in the T cell by at least 50%, at least 60%, at least 70%, at least 80%, at least 90% or more, or (c) modification of the T cell such that CD7 and TCRβ is not expressed (e.g., by deletion or disruption of the gene encoding CD7 and / or TCRβ. In further embodiments, the T cell comprises a suicide gene. In non-limiting examples, a protein coding sequence of a modified Human-Herpes Simplex Virus-1-thymidine kinase (TK) gene is fused in-frame to the extracellular and transmembrane domains of the human CD34 cDNA is expressed in the **CD7*TCRβ**-dCARTΔCD7ΔTCRβ cells.

In another embodiment, the disclosure provides an engineered T cell comprising a dCAR that specifically binds CD7 and CD2, wherein the T cell is deficient in CD7 and CD2, e.g., **CD7*CD2**-dCARTΔCD7ΔCD2 cell. In non-limiting examples the deficiency in CD7 and CD2 resulted from (a) modification of CD7 and CD2 expressed by the T cell such that the chimeric antigen receptor no longer specifically binds the modified CD7 and CD2, (b) modification of the T cell such that expression of the CD7 and CD2 is reduced in the T cell by at least 50%, at least 60%, at least 70%, at least 80%, at least 90% or more, or (c) modification of the T cell such that CD7 and CD2 is not expressed (e.g., by deletion or disruption of the gene encoding CD7 and / or CD2. In further embodiments, the T cell comprises a suicide gene. In non-limiting examples, a protein coding sequence of a modified Human-Herpes Simplex Virus-1-thymidine kinase (TK) gene is fused in-frame to the extracellular and transmembrane domains of the human CD34 cDNA and is expressed in **CD7*CD2**-dCARTΔCD7ΔCD2 cells.

In another embodiment, the disclosure provides an engineered T cell comprising a dCAR that specifically binds CD7 and CD5, wherein the T cell is deficient in CD7 and CD5, e.g., **CD7*CD5**-dCARTΔCD7ΔCD5 cell. In non-limiting examples the deficiency in CD7 and CD5 resulted from (a) modification of CD7 and CD5 expressed by the T cell such that the chimeric antigen receptor no longer specifically binds the modified CD7 and CD5, (b) modification of the T cell such that expression of the CD7 and CD5 is reduced in the T cell by at least 50%, at least 60%, at least 70%, at least 80%, at least 90% or more, or (c) modification of the T cell such that CD7 and CD5 is not expressed (e.g., by deletion or disruption of the gene encoding CD7 and / or CD5. In further embodiments, the T cell comprises a suicide gene. In non-limiting examples, a protein coding sequence of a modified Human-Herpes Simplex Virus-1-thymidine kinase (TK) gene is fused in-frame to the extracellular and transmembrane domains of the human CD34 cDNA and is expressed in CD7*CD5-dCARTΔCD7ΔCD5 cells.

In another embodiment, the disclosure provides an engineered T cell comprising a dCAR that specifically binds CD7 and CD4, wherein the T cell is deficient in CD7 and CD4 (e.g., **CD7*CD4**-dCARTΔCD7ΔCD4 cell). In non-limiting examples the deficiency in CD7 and CD4 resulted from (a) modification of CD7 and CD4 expressed by the T cell such that the chimeric antigen receptor no longer specifically binds the modified CD7 and CD4, (b) modification of the T cell such that expression of the CD7 and CD4 is reduced in the T cell by at least 50%, at least 60%, at least 70%, at least 80%, at least 90% or more, or (c) modification of the T cell such that CD7 and CD4 is not expressed (e.g., by deletion or disruption of the gene encoding CD7 and / or CD4. In further embodiments, the T cell comprises a suicide gene. In non-limiting examples the suicide gene expressed in the **CD7*CD4**-dCARTΔCD7ΔCD4 cells encodes a modified Human-Herpes Simplex Virus-1-thymidine kinase (TK) gene is fused in-frame to the extracellular and transmembrane domains of the human CD34 cDNA and is expressed in **CD7*CD4**-dCARTΔCD7ΔCD4 cells.

In one embodiment, a dual CAR-T cell comprises (i) a first chimeric antigen receptor (CAR) polypeptide comprising a first signal peptide, a first extracellular ligand-binding domain, , a first hinge region, a first transmembrane domain, one or more co-stimulatory domains, and a first signaling transducing domain; and (ii) a second chimeric antigen receptor polypeptide comprising a second signaling peptide, a second extracellular ligand-binding domain, a second hinge region, a second transmembrane domain, one or more co-stimulatory domains, and a second signaling transducing domain; wherein the first extracellular ligand-binding domain and the second extracellular ligand-binding domain have affinities for different cell surface molecules; and wherein the dual CAR-T cell possesses one or more genetic disruptions resulting in reduced expression of the cell surface molecule in the dual CAR-T cell.

In a second embodiment, the first and/or second signal peptide is a CD8α signal sequence.

In a third embodiment, the first and/or second extracellular ligand-binding domain is a fusion protein of the variable regions of immunoglobulin heavy and light chains, designated V_{H}1 and V_{L}1 or V_{H}2 and V_{L}2, and connected by a short linker peptide of 5 amino acids (GGGGS). In some embodiments, this linker peptide is repeated 3 or 4 times. In some embodiments, the first and/or second antigen recognition domain can be selected from V_{H}1 - (GGGGS)₃₋₄ - V_{L}1 or V_{L}1 - (GGGGS)₃₋₄ - V_{H}1 or V_{H}2 - (GGGGS)₃₋₄ - V_{L}2 or V_{L}2 - (GGGGS)₃₋₄ - V_{H}2.

In another embodiment, the first and/or second hinge region comprises CD8α.

In another embodiment, the first and/or second transmembrane domain is CD8 or CD28.

In some embodiments, the first and/or second co-stimulatory domain comprises 4-1BB, CD28, or a combination of both, in either order, i.e., 4-1BB-CD28 or CD28-4-1BB.

In some embodiments, the first and/or second signaling domain is CD3ζ or a CD3ζ bi-peptide., i.e. CD3ζ - CD3ζ.

In some embodiments, the CAR polypeptide comprises at least one high efficiency cleavage site, wherein the high efficiency cleavage site is selected from P2A, T2A, E2A, and F2A.

In some embodiments, the CAR polypeptide comprises a suicide gene.

In some embodiments, the CAR polypeptide comprises a mutant cytokine receptor.

In some embodiments, the dual CAR-T cell targets two antigens selected from CD7 and one of CD5, CD2, CD4, CD3, CD33, CD123 (IL3RA), CD371 (CLL-1; CLEC12A), CD117 (c-kit), CD135 (FLT3), BCMA, CS1, CD38, CD79A, CD79B, CD138, and CD19, APRIL, and TACI.

Additional examples of dual CARs are given below in Table 5.

**Table 5. Dual CARs and dCAR-Ts**

| **Example** | **Antigen Targets of CARs in dCAR-T cell** | **Antigen Deletion/ Suppression** |
|---|---|---|
| **D43** | CD2xCD7 | CD7 |
| **D44** | CD2xCD7 | CD2 and CD7 |
| **D46** | CD2xCD7 | CD7 and TRAC |
| **D47** | CD2xCD7 | CD2 and CD7 and TRAC |
| **D58** | CD3εxCD7 | CD7 |
| **D59** | CD3εxCD7 | CD3ε and CD7 |
| **D69** | CD4xCD7 | CD7 |
| **D70** | CD4xCD7 | CD4 and CD7 |
| **D72** | CD4xCD7 | CD7 and TRAC |
| **D73** | CD4xCD7 | CD4 and CD7 and TRAC |
| **D76** | CD5xCD7 | CD7 |
| **D77** | CD5xCD7 | CD5 and CD7 |
| **D79** | CD5xCD7 | CD7 and TRAC |
| **D80** | CD5xCD7 | CD5 and CD7 and TRAC |
| **D121** | TCRβxCD7 | CD7 |
| **D122** | TCRβxCD7 | TCRβ and CD7 |
| **D141** | TRACxCD7 | CD7 |
| **D142** | TRACxCD7 | TRAC and CD7 |

### Tandem CAR-T Cells (tCAR-T)

Tandem CAR-T cells are not according to the claimed invention. A tandem CAR-T cell (tCAR-T), is a T cell with a single chimeric antigen polypeptide comprising two distinct extracellular ligand-binding domains capable of interacting with two different cell surface molecules, wherein the extracellular ligand-binding domains are linked together by a flexible linker and share one or more costimulatory domains, wherein the binding of the first or the second extracellular ligand-binding domain will signal through one or more the costimulatory domains and a signaling transducing domain.

In a second embodiment, an engineered T cell comprises a tandem Chimeric Antigen Receptor (tCAR), wherein one extracellular ligand-binding domain specifically binds CD7 and the second extracellular ligand-binding domain binds the TCR receptor alpha chain (TRAC), wherein the T cell is deficient in CD7 and TRAC, e.g., **CD7*TRAC**-tCARTΔCD7ΔTRAC cell. In non-limiting examples the deficiency in CD7 and the TCR receptor alpha chain (TRAC) resulted from (a) modification of CD7 and the TCR receptor alpha chain (TRAC) expressed by the T cell such that the tCAR no longer specifically binds the modified CD7 and the TCR receptor alpha chain (TRAC), (b) modification of the T cell such that expression of the CD7 and the TCR receptor alpha chain (TRAC) is reduced in the T cell by at least 50%, at least 60%, at least 70%, at least 80%, at least 90% or more, or (c) modification of the T cell such that CD7 and the TCR receptor alpha chain (TRAC) is not expressed (e.g., by deletion or disruption of the gene encoding CD7 and / or the TCR receptor alpha chain (TRAC). In further embodiments, the T cell comprises a suicide gene. In non-limiting examples, a protein-coding sequence of a modified Human-Herpes Simplex Virus-1-thymidine kinase (TK) gene is fused in-frame to the extracellular and transmembrane domains of the human CD34 cDNA and is expressed in the **CD7*TRAC**-tCARTΔCD7ΔTRAC cells.

In another embodiment, an engineered T cell comprises a tandem Chimeric Antigen Receptor (tCAR), wherein one extracellular ligand-binding domain specifically binds CD7 and the second extracellular ligand-binding domain binds the TCRβ chain, wherein the T cell is deficient in CD7 and TCRβ chain, e.g., a **CD7*TCRβ**-tCARTΔCD7ΔTCRβ cell. In non-limiting examples the deficiency in CD7 and the TCRβ chain resulted from (a) modification of CD7 and TCRβ expressed by the T cell such that the tCAR no longer specifically binds the modified CD7 and TCRβ, (b) modification of the T cell such that expression of the CD7 and TCRβ is reduced in the T cell by at least 50%, at least 60%, at least 70%, at least 80%, at least 90% or more, or (c) modification of the T cell such that CD7 and TCRβ is not expressed (e.g., by deletion or disruption of the gene encoding CD7 and / or TCRβ. In further embodiments, the T cell comprises a suicide gene. In non-limiting examples, a protein coding sequence of a modified Human-Herpes Simplex Virus-1-thymidine kinase (TK) gene is fused in-frame to the extracellular and transmembrane domains of the human CD34 cDNA and is expressed in the **CD7*TCRβ**-tCARTΔCD7ΔTCRβ cells.

In another embodiment, an engineered T cell comprises a tandem Chimeric Antigen Receptor (tCAR), wherein one extracellular ligand-binding domain specifically binds CD7 and the second extracellular ligand-binding domain binds CD2, wherein the T cell is deficient in CD7 and CD2, e.g., **CD7*CD2**-tCARTΔCD7ΔCD2 cell. In non-limiting examples the deficiency in CD7 and CD2 resulted from (a) modification of CD7 and CD2 expressed by the T cell such that the tCAR no longer specifically binds the modified CD7 and CD2, (b) modification of the T cell such that expression of the CD7 and CD2 is reduced in the T cell by at least 50%, at least 60%, at least 70%, at least 80%, at least 90% or more, or (c) modification of the T cell such that CD7 and CD2 is not expressed (e.g., by deletion or disruption of the gene encoding CD7 and / or CD2. In further embodiments, the T cell comprises a suicide gene. In non-limiting examples, a protein-coding sequence of a modified Human-Herpes Simplex Virus-1-thymidine kinase (TK) gene is fused in-frame to the extracellular and transmembrane domains of the human CD34 cDNA and is expressed in the CD7*CD2-tCARTΔCD7ΔCD2 cells.

In another embodiment, an engineered T cell comprises a tandem Chimeric Antigen Receptor (tCAR), wherein one extracellular ligand-binding domain specifically binds CD7 and the second extracellular ligand-binding domain binds CD5, wherein the T cell is deficient in CD7 and CD5, e.g., **CD7*CD5**-tCARTΔCD7ΔCD5 cell. In non-limiting examples the deficiency in CD7 and CD5 resulted from (a) modification of CD7 and CD5 expressed by the T cell such that the tCAR no longer specifically binds the modified CD7 and CD5, (b) modification of the T cell such that expression of the CD7 and CD5 is reduced in the T cell by at least 50%, at least 60%, at least 70%, at least 80%, at least 90% or more, or (c) modification of the T cell such that CD7 and CD5 is not expressed (e.g., by deletion or disruption of the gene encoding CD7 and / or CD5. In further embodiments, the T cell comprises a suicide gene. In non-limiting examples, a protein coding sequence of a modified Human-Herpes Simplex Virus-1-thymidine kinase (TK) gene is fused in-frame to the extracellular and transmembrane domains of the human CD34 cDNA and is expressed in the **CD7*CD5**-tCARTΔCD7ΔCD5 cells.

In another embodiment, an engineered T cell comprises a tandem Chimeric Antigen Receptor (tCAR), wherein one extracellular ligand-binding domain specifically binds CD7 and the second extracellular ligand-binding domain binds CD4, wherein the T cell is deficient in CD7 and CD4, e.g., **CD7*CD4**-tCARTΔCD7ΔCD4 cell. In non-limiting examples the deficiency in CD7 and CD4 resulted from (a) modification of CD7 and CD4 expressed by the T cell such that the tCAR no longer specifically binds the modified CD7 and CD4, (b) modification of the T cell such that expression of the CD7 and CD4 is reduced in the T cell by at least 50%, at least 60%, at least 70%, at least 80%, at least 90% or more, or (c) modification of the T cell such that CD7 and CD4 is not expressed (e.g., by deletion or disruption of the gene encoding CD7 and / or CD4. In further embodiments, the T cell comprises a suicide gene. In non-limiting examples, a protein-coding sequence of a modified Human-Herpes Simplex Virus-1-thymidine kinase (TK) gene is fused in-frame to the extracellular and transmembrane domains of the human CD34 cDNA and is expressed in the **CD7*CD4**-tCARTΔCD7ΔCD4 cells.

In another embodiment, a linear tandem CAR-T cell comprises a chimeric antigen receptor (CAR) polypeptide comprising a first signal peptide, a first extracellular ligand-binding domain, a second extracellular ligand-binding domain, a hinge region, a transmembrane domain, one or more co-stimulatory domains, and a signaling transducing domain, wherein the first extracellular ligand-binding antigen recognition domain and the second extracellular ligand-binding antigen recognition domain have affinities for different cell surface molecules, i.e., antigens on a cancer cell, for example, a malignant T cell, malignant B cell, or malignant plasma cell; and wherein the linear tandem CAR-T cell possesses one or more genetic modifications, deletions, or disruptions resulting in reduced expression of the cell surface molecules in the linear tandem CAR-T cell.

In another embodiment, the signal peptide is the signal peptide from human CD8α (SEQ ID NO: 1).

In a third embodiment, the first extracellular ligand-binding domain comprises a single chain antibody fragment (scFv), comprising the light (V_{L}) and the heavy (V_{H}) variable fragment, designated V_{H}1 and V_{L}1 and joined by a linker (e.g., GGGGS). In some embodiments, this linker peptide is repeated 2, 3, 4, 5 or 6 times. In some embodiments, the first antigen recognition domain can be selected from: 1) V_{H}1 - (GGGGS)₃₋₄ - V_{L}1 or 2) V_{L}1 - (GGGGS)₃₋₄ -V_{H}1.

In some embodiments, the second extracellular ligand-binding domain comprises a single chain antibody fragment (scFv), comprising the light (V_{L}) and the heavy (V_{H}) variable fragment, designated V_{H}2 and V_{L}2 and joined by a linker (e.g., GGGGS). In some embodiments, this linker peptide is repeated 2, 3, 4, 5 or 6 times. In some embodiments, the first antigen recognition domain can be selected from: 1) V_{H}2 - (GGGGS)₃₋₄ - V_{L}2 or 2) V_{L}2 - (GGGGS)₃₋₄ -V_{H}2.

In further embodiments, the first antigen recognition domain and second antigen recognition domain are connected by a short linker peptide of 5 amino acids (GGGGS). In some embodiments, this linker peptide is repeated 2, 3, 4, 5 or 6 times.

### Linear Tandem CAR Constructs

In one embodiment of a linear tandem CAR construct, the first extracellular ligand-binding domain comprises a single chain antibody fragment (scFv), comprising the heavy (V_{H}) and the light (V_{L}) variable fragment, designated V_{H}1 and V_{L}1, and joined by a linker (e.g., GGGGS) ₂₋₆. The second extracellular ligand-binding domain antigen recognition comprises a single chain antibody fragment (scFv), comprising the light (V_{L}) and the heavy (V_{H}) variable fragment, designated V_{L}2 and V_{H}2, and joined by a linker (e.g., GGGGS)₂₋₆.

In a second embodiment of a linear tandem CAR construct, the first extracellular ligand-binding domain comprises a single chain antibody fragment (scFv), comprising the heavy (V_{H}) and the light (V_{L}) variable fragment, designated V_{H}2 and V_{L}2, and joined by a linker (e.g., GGGGS)₂₋₆. The second extracellular ligand-binding domain antigen recognition comprises a single chain antibody fragment (scFv), comprising the light (V_{L}) and the heavy (V_{H}) variable fragment, designated V_{L}1 and V_{H}1, and joined by a linker (e.g., GGGGS)₂₋₆.

In a third embodiment of a linear tandem CAR construct, the first extracellular ligand-binding domain comprises a single chain antibody fragment (scFv), comprising the heavy (VL) and the light (VH) variable fragment, designated V_{L}1 and V_{H}1, and joined by a linker (e.g., GGGGS)₂₋₆. The second extracellular ligand-binding domain antigen recognition comprises a single chain antibody fragment (scFv), comprising the light (V_{H}) and the heavy (V_{L}) variable fragment, designated V_{H}2 and V_{L}2, and joined by a linker (e.g., GGGGS)₂₋₆.

In a fourth embodiment of a linear tandem CAR construct, the first extracellular ligand-binding domain comprises a single chain antibody fragment (scFv), comprising the heavy (V_{L}) and the light (V_{H}) variable fragment, designated V_{L}2 and V_{H}2, and joined by a linker (e.g., GGGGS)₂₋₆. The second extracellular ligand-binding domain antigen recognition comprises a single chain antibody fragment (scFv), comprising the light (V_{H}) and the heavy (V_{L}) variable fragment, designated V_{H}1 and V_{L}1, and joined by a linker (e.g., GGGGS)₂₋₆.

For each of the linear tandem CAR construct embodiments, the first and second extracellular ligand-binding domains targets a surface molecule, i.e., an antigen expressed on a malignant T cell is selected from CD7 and one of, but not limited to, BCMA, CS1, CD38, CD138, CD19, CD33, CD123, CD371, CD117, CD135, Tim-3, CD5, CD2, CD4, CD3, CD79A, CD79B, APRIL, CD56, and CD1a.

Further examples of linear tandem CARs are given below in Table 6.

**Table 6. Tandem CARs and CAR-Ts (Linear or Hairpin).**

| **Example** | **Antigen Target CAR-T cell** | **Antigen Deletion/ Suppression** |
|---|---|---|
| **T43** | CD2xCD7 | CD7 |
| **T44** | CD2xCD7 | CD2 and CD7 |
| **T46** | CD2xCD7 | CD7 and TRAC |
| **T47** | CD2xCD7 | CD2 and CD7 and TRAC |
| **T58** | CD3εxCD7 | CD7 |
| **T59** | CD3εxCD7 | CD3ε and CD7 |
| **T69** | CD4xCD7 | CD7 |
| **T70** | CD4xCD7 | CD4 and CD7 |
| **T73** | CD4xCD7 | CD4 and CD7 and TRAC |
| **T76** | CD5xCD7 | CD7 |
| **T77** | CD5xCD7 | CD5 and CD7 |
| **T79** | CD5xCD7 | CD7 and TRAC |
| **T80** | CD5xCD7 | CD5 and CD7 and TRAC |
| **T121** | TCRβxCD7 | CD7 |
| **T122** | TCRβxCD7 | TCRβ and CD7 |
| **T141** | TRACxCD7 | CD7 |
| **T142** | TRACxCD7 | TRAC and CD7 |

For example, provided herein are linear tandem CAR constructs which may incorporate the V_{H} and V_{L} domains of scFvs targeting any of the antigen pairs provided in Table 6 above.

**Table 7. Linear Tandem CAR Constructs.**

| **7-I** | **7-II** | **7-III** | **7-IV** | **7-V** | **7-VI** | **7-VII** | **7-VIII** |
|---|---|---|---|---|---|---|---|
| CD8a | CD8a | CD8a | CD8a | CD8a | CD8a | CD8a | CD8a |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |
| | | | | | | | |

| **7-IX** | **7-X** | **7-XI** | **7-XII** | **7-XIII** | **7-XIV** | **7-XV** | **7-XVI** |
|---|---|---|---|---|---|---|---|
| CD8a | CD8a | CD8a | CD8a | CD8a | CD8a | CD8a | CD8a |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |
| **V_{L}1** | **V_{L}1** | **V_{L}1** | **V_{L}1** | **V_{L}1** | **V_{L}1** | **V_{L}1** | **V_{L}1** |
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |
| | | | | | | | |

| **7-XVII** | **7-XVIII** | **7-XIX** | **7-XX** | **7-XXI** | **7-XXII** | **7-XXIII** | **7-XIV** |
|---|---|---|---|---|---|---|---|
| CD8a | CD8a | CD8a | CD8a | CD8a | CD8a | CD8a | CD8a |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |
| | | | | | | | |

| **7-XXV** | **7-XXVI** | **7-XXVII** | **7-XXVIII** | **7-XIX** | **7-XXX** | **7-XXXI** | **7-XXXII** |
|---|---|---|---|---|---|---|---|
| CD8a | CD8a | CD8a | CD8a | CD8a | CD8a | CD8a | CD8a |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |

### Hairpin Tandem CAR Constructs

In one embodiment of a hairpin tandem CAR construct, the first extracellular ligand-binding domain comprises a single chain antibody fragment (scFv), comprising two heavy chain variable fragments, designated V_{H}1 and V_{H}2, and joined by a linker (e.g., GGGGS)₂₋₆. The second extracellular ligand-binding domain antigen recognition comprises a single chain antibody fragment (scFv), comprising two light chain variable fragments, designated V_{L}2 and V_{L}1, and joined by a linker (e.g., GGGGS)₂₋₆.

In a second embodiment of a hairpin tandem CAR construct, the first extracellular ligand-binding domain comprises a single chain antibody fragment (scFv), comprising two heavy chain variable fragments, designated V_{H}2 and V_{H}1, and joined by a linker (e.g., GGGGS)₂₋₆. The second extracellular ligand-binding domain antigen recognition comprises a single chain antibody fragment (scFv), comprising two light chain variable fragments, designated V_{L}1 and V_{L}2, and joined by a linker (e.g., GGGGS)₂₋₆.

In a third embodiment of a hairpin tandem CAR construct, the first extracellular ligand-binding domain comprises a single chain antibody fragment (scFv), comprising two light chain variable fragments, designated V_{L}1 and V_{L}2, and joined by a linker (e.g., GGGGS)₂₋₆. The second extracellular ligand-binding domain antigen recognition comprises a single chain antibody fragment (scFv), comprising two heavy chain variable fragments, designated V_{H}2 and V_{H}1, and joined by a linker (e.g., GGGGS)₂₋₆.

In a fourth embodiment of a hairpin tandem CAR construct, the first extracellular ligand-binding domain comprises a single chain antibody fragment (scFv), comprising two light chain variable fragments, designated V_{L}2 and V_{L}1, and joined by a linker (e.g., GGGGS)₂₋₆. The second extracellular ligand-binding domain antigen recognition comprises a single chain antibody fragment (scFv), comprising two heavy chain variable fragments, designated V_{H}1 and V_{H}2, and joined by a linker (e.g., GGGGS)₂₋₆.

For each of the hairpin tandem CAR construct embodiments, the first and second extracellular ligand-binding domains targets a surface molecule, i.e., an antigen expressed on a malignant T cell is selected from CD7 and one of, but not limited to, BCMA, CS1, CD38, CD138, CD19, CD33, CD123, CD371, CD117, CD135, Tim-3, CD5, CD2, CD4, CD3, CD79A, CD79B, APRIL, CD56, and CD1a.

Additional examples of hairpin tandem CARs are given above in Table 6.

Furthermore, provided herein are CAR constructs and CAR- T cells which may incorporate the V_{H} and V_{L} domains of scFvs targeting CD2 and CD7.

Additionally, provided herein are hairpin tandem CAR constructs which may incorporate the V_{H} and V_{L} domains of scFvs targeting any of the antigen pairs provided in Table 6.

**Table 9. Hairpin Tandem CAR Constructs.**

| **9-I** | **9-II** | **9-III** | **9-IV** | **9-V** | **9-VI** | **9-VII** | **9-VIII** |
|---|---|---|---|---|---|---|---|
| CD8a | CD8a | CD8a | CD8a | CD8a | CD8a | CD8a | CD8a |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |
| | | | | | | | |

| **9-IX** | **9-X** | **9-XI** | **9-XII** | **9-XIII** | **9-XIV** | **9-XV** | **9-XVI** |
|---|---|---|---|---|---|---|---|
| CD8a | CD8a | CD8a | CD8a | CD8a | CD8a | CD8a | CD8a |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |
| | | | | | | | |

| **9-XVII** | **9-XVIII** | **9-XIX** | **9-XX** | **9-XXI** | **9-XXII** | **9-XXIII** | **9-XIV** |
|---|---|---|---|---|---|---|---|
| CD8a | CD8a | CD8a | CD8a | CD8a | CD8a | CD8a | CD8a |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |
| | | | | | | | |

| **9-XXV** | **9-XXVI** | **9-XXVII** | **9-XXVIII** | **9-XIX** | **9-XXX** | **9-XXXI** | **9-XXXII** |
|---|---|---|---|---|---|---|---|
| CD8a | CD8a | CD8a | CD8a | CD8a | CD8a | CD8a | CD8a |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |

Also provided herein in Table 11 are hairpin tandem CAR constructs with a (Cys = Cys) double-stranded bond (DSB) which may incorporate the V_{H} and V_{L} domains of scFvs targeting any of the antigen pairs provided in Table 6.

**Table 11. Hairpin Tandem DSB CAR Constructs with a (Cys=Cys) Double-Stranded Bond (DSB).**

| **11-I** | **11-II** | **11-III** | **11-IV** | **11-V** | **11-VI** | **11-VII** | **11-VIII** |
|---|---|---|---|---|---|---|---|
| CD8a | CD8a | CD8a | CD8a | CD8a | CD8a | CD8a | CD8a |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₀₋₁₎GGGGC ₍₁₎GGGGS | GGGGS₍₀₋₁₎GGGGC ₍₁₎GGGGS | GGGGS₍₀₋₁₎GGGGC ₍₁₎GGGGS | GGGGS₍₀₋₁₎GGGGC ₍₁₎GGGGS | GGGGS₍₀₋₁₎GGGGC ₍₁₎GGGGS | GGGGS₍₀₋₁₎GGGGC ₍₁₎GGGGS | GGGGS₍₀₋₁₎GGGGC ₍₁₎GGGGS | GGGGS₍₀₋₁₎GGGGC ₍₁₎GGGGS |
| ₍₁₋₂₎ | ₍₁₋₂₎ | ₍₁₋₂₎ | ₍₁₋₂₎ | ₍₁₋₂₎ | ₍₁₋₂₎ | ₍₁₋₂₎ | ₍₁₋₂₎ |
| GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ |
| GGGGC ₍₁₎GGGGS ₍₀₋₁₎ | GGGGC (1)GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ |
| | | | | | | | |

| **11-IX** | **11-X** | **11-XI** | **11-XII** | **11-XIII** | **11-XIV** | **11-XV** | **11-XVI** |
|---|---|---|---|---|---|---|---|
| CD8a | CD8a | CD8a | CD8a | CD8a | CD8a | CD8a | CD8a |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| V_{H}1 | V_{H}1 | V_{H}1 | V_{H}1 | V_{H}1 | V_{H}1 | V_{H}1 | V_{H}1 |
|---|---|---|---|---|---|---|---|
| GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ |
| GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ |
| GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3z₍₁₋₂₎ | CD3z₍₁₋₂₎ | CD3z₍₁₋₂₎ | CD3z₍₁₋₂₎ | CD3z₍₁₋₂₎ | CD3z₍₁₋₂₎ | CD3z₍₁₋₂₎ | CD3z₍₁₋₂₎ |
| | | | | | | | |

| **11-XVII** | **11-XVIII** | **11-XIX** | **11-XX** | **11-XXI** | **11-XXII** | **11-XXIII** | **11-XXIV** |
|---|---|---|---|---|---|---|---|
| CD8a | CD8a | CD8a | CD8a | CD8a | CD8a | CD8a | CD8a |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ |
| GGGGP ₍₁₎GGGGS (2-3) | GGGGP ₍₁₎GGGGS (2-3) | GGGGP ₍₁₎GGGGS (2-3) | GGGGP ₍₁₎GGGGS (2-3) | GGGGP ₍₁₎GGGGS (2-3) | GGGGP ₍₁₎GGGGS (2-3) | GGGGP ₍₁₎GGGGS (2-3) | GGGGP ₍₁₎GGGGS (2-3) |
| GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎ | CD3ζ₍₁₋₂₎₎ |
| | | | | | | | |

| **11-XXV** | **11-XXVI** | **11-XXVII** | **11-XXVIII** | **11-XXIX** | **11-XXX** | **11-XXXI** | **11-XXXII** |
|---|---|---|---|---|---|---|---|
| CD8a | CD8a | CD8a | CD8a | CD8a | CD8a | CD8a | CD8a |

| **V_{L}2** | **V_{L}2** | **V_{L}2** | **V_{L}2** | **V_{L}2** | **V_{L}2** | **V_{L}2** | **V_{L}2** |
|---|---|---|---|---|---|---|---|
| GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ | GGGGS₍₂₋₆₎ |

| **V_{L}1** | **V_{L}1** | **V_{L}1** | **V_{L}1** | **V_{L}1** | **V_{L}1** | **V_{L}1** | **V_{L}1** |
|---|---|---|---|---|---|---|---|
| GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ | GGGGS₍₀₋₁₎GGGGC ₍₁₎GGGGS ₍₁₋₂₎ |
| GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ | GGGGP ₍₁₎GGGGS ₍₂₋₃₎ |
| GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ | GGGGC ₍₁₎GGGGS ₍₀₋₁₎ |

| **V_{H}1** | **V_{H}1** | **V_{H}1** | **V_{H}1** | **V_{H}1** | **V_{H}1** | **V_{H}1** | **V_{H}1** |
|---|---|---|---|---|---|---|---|
| GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ | GGGGS₍₃₋₄₎ |

| **V_{H}2** | **V_{H}2** | **V_{H}2** | **V_{H}2** | **V_{H}2** | **V_{H}2** | **V_{H}2** | **V_{H}2** |
|---|---|---|---|---|---|---|---|
| CD8 Tm | CD8 Tm | CD8 Tm | CD8 Tm | CD28 Tm | CD28 Tm | CD28 Tm | CD28 Tm |
| 41BB | CD28 | 41BB - CD28 | CD28 - 41BB | 41BB | CD28 | 41BB - CD28 | CD28 - 41BB |
| CD3z₍₁₋₂₎ | CD3z₍₁₋₂₎ | CD3z₍₁₋₂₎ | CD3z₍₁₋₂₎ | CD3z₍₁₋₂₎ | CD3z₍₁₋₂₎ | CD3z₍₁₋₂₎ | CD3z₍₁₋₂₎ |

### Methods for Engineering CARs in a Dual or Tandem Construction with Gene Editing

In a further aspect, a CAR-T cell control may be created. For example, the control CAR-T cell may include an extracellular domain that binds to an antigen not expressed on a malignant T-cell. For example, if the therapeutic CAR-T cell targets a T-cell antigen such as CD7, or multiple T cell antigens, the antigen the control CAR-T cell binds to may be CD19. CD19 is an antigen expressed on B cells but not on T cells, so a CAR-T cell with an extracellular domain adapted to bind to CD19 will not bind to T cells. These CAR-T cells may be used as controls to analyze the binding efficiencies and non-specific binding of CAR-T cells targeted to the cancer of interest and/or recognizing the antigen of interest.

CARs may be further designed as disclosed in WO2018027036A1, optionally employing variations which will be known to those of skill in the art. Lentiviral vectors and cell lines can be obtained, and guide RNAs designed, validated, and synthesized, as disclosed therein as well as by methods known in the art and from commercial sources.

Engineered CARs may be introduced into T cells using retroviruses, which efficiently and stably integrate a nucleic acid sequence encoding the chimeric antigen receptor into the target cell genome. Other methods known in the art include, but are not limited to, lentiviral transduction, transposon-based systems, direct RNA transfection, and CRISPR/Cas systems (e.g., type I, type II, or type Ill systems using a suitable Cas protein such Cas3, Cas4, Cas5, Cas5e (or CasD), Cas6, Cas6e, Cas6f, Cas7, Cas8a1 , Cas8a2, Cas8b, Cas8c, Cas9, Cas10, Casl Od, CasF, CasG, CasH, Csy1 , Csy2, Csy3, Cse1 (or CasA), Cse2 (or CasB), Cse3 (or CasE), Cse4 (or CasC), Csc1 , Csc2, Csa5, Csn2, Csm2, Csm3, Csm4, Csm5, Csm6, Cmr1 , Cmr3, Cmr4, Cmr5, Cmr6, Csb1 , Csb2, Csb3,Csx17, Csx14, Csx1 0, Csx16, CsaX, Csx3, Csz1 , Csx15, Csf1 , Csf2, Csf3, Csf4, and Cu1966, etc.). Zinc finger nucleases (ZFNs) and transcription activator-like effector nucleases (TALENs) may also be used. See, e.g., Shearer RF and Saunders DN, "Experimental design for stable genetic manipulation in mammalian cell lines: lentivirus and alternatives," Genes Cells 2015 Jan; 20(1): 1-10.

Manipulation of PI3K signaling can be used to prevent altered CAR-T cell differentiation due to constitutive CAR self-signaling and foster long-lived memory T cell development. pharmacologic blockade of PI3K during CAR-T manufacture and ex vivo expansion can abrogate preferential effector T cell development and restore CAR-T effector/memory ratio to that observed in empty vector transduced T cells, which can improve *in vivo* T cell persistence and therapeutic activity. Inhibition of p1106 PI3K can enhance efficacy and memory in tumor-specific therapeutic CD8 T cells, while inhibition of p110α PI3K can increase cytokine production and antitumor response.
This is proposed to be because the presence of a CAR on a T cell's surface can alter its activation and differentiation, even in the absence of ligand. Constitutive self-signaling through CAR, related to both the scFv framework and the signaling domains, can lead to aberrant T cell behavior, including altered differentiation and decreased survival. This is significant as the effectiveness of CAR-T cells in patients is directly associated with their in vivo longevity. The presence of the CD28 costimulatory domain increased CAR-T cell exhaustion induced by persistent CAR self-signaling; the 4-1BB costimulatory domain had a lesser effect. Furthermore, CD3-zeta significantly enhances the constitutive activation of the PI3K, AKT, mTOR, and glycolysis pathways, and fostered formation of short-lived effector cells over central/stem memory cells. See, e.g., Zhang W. et al., "Modulation of PI3K signaling to improve CAR T cell function," Oncotarget, 2018 Nov 9; 9(88): 35807-35808.

### Cytokine Gene Deletion or Suppression

In addition to gene-editing the TCR and cell surface proteins and antigens, genes for secretable proteins such as cytokines and chemokines may be edited. Such editing would be done, e.g., to reduce or prevent the development or maintenance of cytokine release syndrome (CRS). CRS is caused by a large, rapid release of cytokines from immune cells in response to immunotherapy (or other immunological stimulus). Modifying, disrupting, or deleting one or more cytokine or chemokine genes can be accomplished using the methods known in the art, such as genetic ablation (gene silencing) in which gene expression is abolished through the alteration or deletion of genetic sequence information. This can be accomplished using known genetic engineering tools in the art, such as Transcription Activator-like Effector Nucleases (TALENs), Zinc Finger Nucleases (ZFNs), CRISPR, by transduction of small hairpin RNAs (shRNAs), by targeted transduction of a CAR into the gene sequence of the cytokine, and the like.

Cytokines or chemokines that can be deleted from immune effector cells as disclosed herein, e.g., using Cas9-CRISPR or by targeted transduction of a CAR into the gene sequence of the cytokine, include without limitation the following: XCL1, XCL2, CCL1, CCL2, CCL3, CCL4, CCL5, CCL7, CCL8, CCL11, CCL13, CCL14, CCL15, CCL16, CCL17, CCL18, CCL19, CCL20, CCL21, CCL22, CCL23, CCL24, CCL25, CCL26, CCL27, CXCL1, CXCL2, CXCL3, CXCL4, CXCL5, CXCL6, CXCL7, CXCL8, CXCL9, CXCL10, CXCL11, CXCL12, CXCL13, CXCL14, CX3CL1, IL-1α, IL-1β, IL-1RA, IL-18, IL-2, IL-4, IL-7, IL-9, IL-13, IL-15, IL-3, IL-5, GM-CSF, IL-6, IL-11, G-CSF, IL-12, LIF, OSM, IL-10, IL-20, IL-14, IL-16, IL-17, IFN-α, IFN-β, IFN-γ, CD154, LT-β, TNF-α ,TNF-β, 4-1BBL, APRIL, CD70, CD153, CD178, GITRL, LIGHT, OX40L, TALL-1, TRAIL, TWEAK, TRANCE, TGF-β1, TGF-β2, TGF-β3, Epo, Tpo, Flt-3L, SCF, M-CSF, MSP, A2M, ACKR1, ACKR2, ACKR3, ACVR1, ACVR2B, ACVRL1, ADIPOQ, AGER, AGRN, AIMP1, AREG, BMP1, BMP10, BMP15, BMP2, BMP3, BMP4, BMP5, BMP6, BMP7, BMP8A, BMP8B, BMPR2, C10orf99, C1QTNF4, C5, CCL28, CCR1, CCR2, CCR3, CCR5, CCR6, CCR7, CD109, CD36, CD4, CD40LG, CD74, CER1, CHRD, CKLF, CLCF1, CMTM1, CMTM2, CMTM3, CMTM4, CMTM5, CMTM6, CMTM7, CMTM8, CNTF, CNTFR, COPS5, CRLF1, CSF1, CSF1R, CSF2, CSF3, CSF3R, CTF1, CX3CR1, CXCL16, CXCL17, CXCR1, CXCR2, CXCR3, CXCR4, CXCR6, EBI3, EDN1, ELANE, ENG, FAM3B, FAM3C, FAM3D, FAS, FASLG, FGF2, FLT3LG, FZD4, GBP1, GDF1, GDF10, GDF11, GDF15, GDF2, GDF3, GDF5, GDF6, GDF7, GDF9, GPI, GREM1, GREM2, GRN, HAX1, HFE2, HMGB1, HYAL2, IFNA10, IFNA14, IFNA16, IFNA2, IFNA5, IFNA6, IFNA8, IFNAR1, IFNAR2, IFNB1, IFNE, IFNG, IFNGR1, IFNK, IFNL1, IFNL3, IFNW1, IL10RA, IL11RA, IL12A, IL12B, IL12RB1, IL17A, IL17B, IL17C, IL17D, IL17F, IL18BP, IL-19, IL1F10, IL1R1, IL1R2, IL1RAPL1, IL1RL1, IL1RN, IL20RA, IL20RB, IL21, IL22, IL22RA1, IL22RA2, IL23A, IL23R, IL24, IL25, IL26, IL27, IL2RA, IL2RB, IL2RG, IL31, IL31RA, IL32, IL33, IL34, IL36A, IL36B, IL36G, IL36RN, IL37, IL6R, IL6ST, INHA, INHBA, INHBB, INHBC, INHBE, ITGA4, ITGAV, ITGB1, ITGB3, KIT, KITLG, KLHL20, LEFTY1, LEFTY2, LIFR, LTA, LTB, LTBP1, LTBP3, LTBP4, MIF, MINOS1-, MSTN, NAMPT, NBL1, NDP, NLRP7, NODAL, NOG, NRG1, NRP1, NRP2, OSMR, PARK7, PDPN, PF4, PF4V1, PGLYRP1, PLP2, PPBP, PXDN, SCG2, SCGB3A1, SECTM1, SLURP1, SOSTDC1, SP100, SPP1, TCAP, TGFBR1, TGFBR2, TGFBR3, THBS1, THNSL2, THPO, TIMP1, TNF, TNFRSF11, TNFRSF1A, TNFRSF9, TNFRSF10, TNFSF11, TNFSF12, TNFSF12-, TNFSF13, TNFSF13B, TNFSF14, TNFSF15, TNFSF18, TNFSF4, TNFSF8, TNFSF9, TRIM16, TSLP, TWSG1, TXLNA, VASN, VEGFA, VSTM1, WFIKKN1, WFIKKN2, WNT1, WNT2, WNT5A, WNT7A, and ZFP36.

The sequences of these genes are known and available in the art.

### Indications and Standards of Care in ACT (CAR-T) Therapy

In some embodiment, the CAR-T cells disclosed herein, and/or generated using the methods disclosed herein, express one or more chimeric antigen receptors (CARs) and can be used as a medicament, i.e., for the treatment of disease.

Cells disclosed herein, and/or generated using the methods disclosed herein, may be used in immunotherapy and adoptive cell transfer, for the treatment, or the manufacture of a medicament for treatment, of cancers, autoimmune diseases, infectious diseases, and other conditions.

The cancer may be a hematologic malignancy or solid tumor. Hematologic malignancies include leukemias, lymphomas, multiple myeloma, and subtypes thereof. Lymphomas can be classified various ways, often based on the underlying type of malignant cell, including Hodgkin's lymphoma (often cancers of Reed-Sternberg cells, but also sometimes originating in B cells; all other lymphomas are non-Hodgkin's lymphomas), B-cell lymphomas, T-cell lymphomas, mantle cell lymphomas, Burkitt's lymphoma, follicular lymphoma, and others as defined herein and known in the art.

B-cell lymphomas include, but are not limited to, diffuse large B-cell lymphoma (DLBCL), chronic lymphocytic leukemia (CLL) /small lymphocytic lymphoma (SLL) , and others as defined herein and known in the art.

T-cell lymphomas include T-cell acute lymphoblastic leukemia/lymphoma (T-ALL), , peripheral T-cell lymphoma (PTCL), T-cell chronic lymphocytic leukemia (T-CLL)Sezary syndrome, and others as defined herein and known in the art.

Leukemias include Acute myeloid (or myelogenous) leukemia (AML), chronic myeloid (or myelogenous) leukemia (CML), acute lymphocytic (or lymphoblastic) leukemia (ALL), chronic lymphocytic leukemia (CLL) hairy cell leukemia (sometimes classified as a lymphoma) , and others as defined herein and known in the art.

Plasma cell cell malignancies include lymphoplasmacytic lymphoma, plasmacytoma, and multiple myeloma.

In some embodiments, the medicament can be used for treating cancer in a patient, particularly for the treatment of solid tumors such as melanomas, neuroblastomas, gliomas or carcinomas such as tumors of the brain, head and neck, breast, lung (e.g., non-small cell lung cancer, NSCLC), reproductive tract (e.g., ovary), upper digestive tract, pancreas, liver, renal system (e.g., kidneys), bladder, prostate and colorectum.

In another embodiment, the medicament can be used for treating cancer in a patient, particularly for the treatment of hematologic malignancies selected from multiple myeloma and acute myeloid leukemia (AML) and for T-cell malignancies selected from T-cell acute lymphoblastic leukemia (T-ALL), non-Hodgkin's lymphoma, and T-cell chronic lymphocytic leukemia (T-CLL).

In some embodiments, the cells may be used in the treatment of autoimmune diseases such as lupus, autoimmune (rheumatoid) arthritis, multiple sclerosis, transplant rejection, Crohn's disease, ulcerative colitis, dermatitis, and the like. In some embodiments, the cells are chimeric autoantibody receptor T-cells, or CAR-Ts displaying antigens or fragments thereof, instead of antibody fragments; in this version of adoptive cell transfer, the B cells that cause autoimmune diseases will attempt to attack the engineered T cells, which will respond by killing them.

In some embodiments, the cells may be used in the treatment of infectious diseases such as HIV and tuberculosis.

In another embodiment, the CAR-T cells of the present disclosure can undergo robust in vivo T cell expansion and can persist for an extended amount of time.

In some embodiments, the treatment of a patient with CAR-T cells of the present disclosure can be ameliorating, curative or prophylactic. It may be either part of an autologous immunotherapy or part of an allogenic immunotherapy treatment. By autologous, it is meant that cells, cell line or population of cells used for treating patients are originating from said patient or from a Human Leucocyte Antigen (HLA) compatible donor. By allogeneic, is meant that the cells or population of cells used for treating patients are not originating from the patient but from a donor.

The treatment of cancer with CAR-T cells of the present disclosure may be in combination with one or more therapies selected from antibody therapy, chemotherapy, cytokine therapy, dendritic cell therapy, gene therapy, hormone therapy, radiotherapy, laser light therapy, and radiation therapy.

The administration of CAR-T cells or a population of CAR-T cells of the present disclosure of the present disclosure be carried out by aerosol inhalation, injection, ingestion, transfusion, implantation or transplantation. The CAR-T cells compositions described herein, i.e., mono CAR, dual CAR, tandem CARs, may be administered to a patient subcutaneously, intradermally, intratumorally, intranodally, intramedullary, intramuscularly, by intravenous or intralymphatic injection, or intraperitoneally. In one embodiment, the cell compositions of the present disclosure are preferably administered by intravenous injection.

The administration of CAR-T cells or a population of CAR-T cells can consist of the administration of 10⁴-10⁹ cells per kg body weight, preferably 10⁵ to 10⁶ cells/kg body weight including all integer values of cell numbers within those ranges. The CAR-T cells or a population of CAR-T cells can be administrated in one or more doses. In another embodiment, the effective amount of CAR-T cells or a population of CAR-T cells are administrated as a single dose. In another embodiment, the effective amount of cells are administered as more than one dose over a period time. Timing of administration is within the judgment of a health care provider and depends on the clinical condition of the patient. The CAR-T cells or a population of CAR-T cells may be obtained from any source, such as a blood bank or a donor. While the needs of a patient vary, determination of optimal ranges of effective amounts of a given CAR-T cell population(s) for a particular disease or conditions are within the skill of the art. An effective amount means an amount which provides a therapeutic or prophylactic benefit. The dosage administered will be dependent upon the age, health and weight of the patient recipient, type of concurrent treatment, if any, frequency of treatment, and the nature of the effect desired.

In another embodiment, the effective amount of CAR-T cells or a population of CAR-T cells or composition comprising those CAR-T cells are administered parenterally. The administration can be an intravenous administration. The administration of CAR-T cells or a population of CAR-T cells or composition comprising those CAR-T cells can be directly done by injection within a tumor.

In one embodiment of the present disclosure, the CAR-T cells or a population of the CAR-T cells are administered to a patient in conjunction with, e.g., before, simultaneously or following, any number of relevant treatment modalities, including but not limited to, treatment with cytokines, or expression of cytokines from within the CAR-T, that enhance T-cell proliferation and persistence and, include but not limited to, IL-2, IL-7, and IL-15 or analogues thereof.

In some embodiments, the CAR-T cells or a population of CAR-T cells of the present disclosure may be used in combination with agents that inhibit immunosuppressive pathways, including but not limited to, inhibitors of TGFβ, interleukin 10 (IL-10), adenosine, VEGF, indoleamine 2,3 dioxygenase 1 (IDO1), indoleamine 2,3-dioxygenase 2 (IDO2), tryptophan 2-3-dioxygenase (TDO), lactate, hypoxia, arginase, and prostaglandin E2.

In another embodiment, the CAR-T cells or a population of CAR-T cells of the present disclosure may be used in combination with T-cell checkpoint inhibitors, including but not limited to, anti-CTLA4 (Ipilimumab) anti-PD1 (Pembrolizumab, Nivolumab, Cemiplimab), anti-PDL1 (Atezolizumab, Avelumab, Durvalumab), anti-PDL2, anti-BTLA, anti-LAG3, anti-TIM3, anti-VISTA, anti-TIGIT, and anti-KIR.

In another embodiment, the CAR-T cells or a population of CAR-T cells of the present disclosure may be used in combination with T cell agonists, including but not limited to, antibodies that stimulate CD28, ICOS, OX-40, CD27, 4-1BB, CD137, GITR, and HVEM.

In another embodiment, the CAR-T cells or a population of CAR-T cells of the present disclosure may be used in combination with therapeutic oncolytic viruses, including but not limited to, retroviruses, picornaviruses, rhabdoviruses, paramyxoviruses, reoviruses, parvoviruses, adenoviruses, herpesviruses, and poxviruses.

In another embodiment, the CAR-T cells or a population of CAR-T cells of the present disclosure may be used in combination with immunostimulatory therapies, such as toll-like receptors agonists, including but not limited to, TLR3, TLR4, TLR7 and TLR9 agonists.

In another embodiment, the CAR-T cells or a population of CAR-T cells of the present disclosure may be used in combination with stimulator of interferon gene (STING) agonists, such as cyclic GMP-AMP synthase (cGAS).

Immune effector cell aplasia, particularly T cell aplasia is also a concern after adoptive cell transfer therapy. When the malignancy treated is a T-cell malignancy, and CAR-T cells target a T cell antigen, normal T cells and their precursors expressing the antigen will become depleted, and the immune system will be compromised. Accordingly, methods for managing these side effects are attendant to therapy. Such methods include selecting and retaining non-malignant T cells or precursors, either autologous or allogeneic (optionally engineered not to cause rejection or be rejected), for later expansion and re-infusion into the patient, after CAR-T cells are exhausted or deactivated. Alternatively, CAR-T cells which recognize and kill subsets of TCR-bearing cells, such as normal and malignant TRBC1⁺, but not TRBC2⁺cells, or alternatively, TRBC2⁺, but not TRBC1⁺cells, may be used to eradicate a T cell malignancy while preserving sufficient normal T cells to maintain normal immune system function.

### Definitions

Unless specifically defined herein, all technical and scientific terms used have the same meaning as commonly understood by a skilled artisan in the fields of gene therapy, biochemistry, genetics, and molecular biology.

As used herein, the terms below have the meanings indicated. When ranges of values are disclosed, and the notation "from n₁ ... to n₂" or "between n₁ ... and n₂" is used, where n₁ and n₂ are the numbers, then unless otherwise specified, this notation is intended to include the numbers themselves and the range between them. This range may be integral or continuous between and including the end values. By way of example, the range "from 2 to 6 carbons" is intended to include two, three, four, five, and six carbons, since carbons come in integer units. Compare, by way of example, the range "from 1 to 3 µM (micromolar)," which is intended to include 1 µM, 3 µM, and everything in between to any number of significant figures (e.g., 1.255 µM, 2.1 µM, 2.9999 µM, etc.).

The term "activation" (and other conjugations thereof) in reference to cells is generally understood to be synonymous with "stimulating" and as used herein refers to treatment of cells that results in expansion of cell populations. In T cells, activation is often accomplished by exposure to CD2 and CD28 (and sometimes CD2 as well) agonists, typically antibodies, optionally coated onto magnetic beads or conjugated to a colloidal polymeric matrix.

The term "antigen" as used herein is a cell surface protein recognized by (i.e., that is the target of) T cell receptor or chimeric antigen receptor. In the classical sense antigens are substances, typically proteins, that are recognized by antibodies, but the definitions overlap insofar as the CAR comprises antibody-derived domains such as light (V_{L}) and heavy (V_{H}) chains recognizing one or more antigen(s).

The term "cancer" refers to a malignancy or abnormal growth of cells in the body. Many different cancers can be characterized or identified by particular cell surface proteins or molecules. Thus, in general terms, cancer in accordance with the present disclosure may refer to any malignancy that may be treated with an immune effector cell, such as a CAR-T cell as described herein, in which the immune effector cell recognizes and binds to the cell surface protein on the cancer cell. As used herein, cancer may refer to a hematologic malignancy, such as multiple myeloma, a T-cell malignancy, or a B cell malignancy. T cell malignancies may include, but are not limited to, T-cell acute lymphoblastic leukemia (T-ALL) or non-Hodgkin's lymphoma. A cancer may also refer to a solid tumor, such as including, but not limited to, cervical cancer, pancreatic cancer, ovarian cancer, mesothelioma, and lung cancer.

A "cell surface protein" as used herein is a protein (or protein complex) expressed by a cell at least in part on the surface of the cell. Examples of cell surface proteins include the TCR (and subunits thereof) and CD7.

A "chimeric antigen receptor" or "CAR" as used herein and generally used in the art, refers to a recombinant fusion protein that has an extracellular ligand-binding domain, a transmembrane domain, and a signaling transducing domain that directs the cell to perform a specialized function upon binding of the extracellular ligand-binding domain to a component present on the target cell. For example, a CAR can have an antibody-based specificity for a desired antigen (e.g., tumor antigen) with a T cell receptor-activating intracellular domain to generate a chimeric protein that exhibits specific anti-target cellular immune activity. First-generation CARs include an extracellular ligand-binding domain and signaling transducing domain, commonly CD3ζ or FcεRIγ. Second generation CARs are built upon first generation CAR constructs by including an intracellular costimulatory domain, commonly 4-1BB or CD28. These costimulatory domains help enhance CAR-T cell cytotoxicity and proliferation compared to first generation CARs. The third generation CARs include multiple costimulatory domains, primarily to increase CAR-T cell proliferation and persistence. Chimeric antigen receptors are distinguished from other antigen binding agents by their ability both to bind MHC-independent antigens and transduce activation signals via their intracellular domain.

A "CAR-bearing immune effector cell" is an immune effector cell which has been transduced with at least one CAR. A "CAR-T cell" is a T cell which has been transduced with at least one CAR; CAR-T cells can be mono, dual, or tandem CAR-T cells. CAR-T cells can be autologous, meaning that they are engineered from a subject's own cells, or allogeneic, meaning that the cells are sourced from a healthy donor, and in many cases, engineered so as not to provoke a host-vs-graft or graft-vs-host reaction. Donor cells may also be sourced from cord blood or generated from induced pluripotent stem cells.

A dual CAR-T cell (dCAR-T), can be defined as a T cell with two distinct chimeric antigen receptor polypeptides with affinity to different target antigen expressed within the same effector cell, wherein each CAR functions independently. The CAR may be expressed from single or multiple polynucleotide sequences.

A tandem CAR-T cell (tCAR-T), can be defined as a T cell with a single chimeric antigen polypeptide containing two distinct extracellular ligand-binding domains with affinity to different targets wherein the extracellular ligand-binding domains are linked through a peptide linker and share one or more common costimulatory domains, wherein binding of either extracellular ligand-binding domain will signal though one or more common costimulatory domains and signal transducing domain.

The term "combination therapy" means the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the conditions or disorders described herein.

The term "composition" as used herein refers to an immunotherapeutic cell population combination with one or more therapeutically acceptable carriers.

The term "disease" as used herein is intended to be generally synonymous, and is used interchangeably with, the terms "disorder," "syndrome," and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The term "fratricide" as used herein means a process which occurs when a CAR-T cell becomes the target of, and is killed by, another CAR-T cell comprising the same chimeric antigen receptor as the target of CAR-T cell, because the targeted cell expresses the antigen specifically recognized by the chimeric antigen receptor on both cells. CAR-T cell comprising a chimeric antigen receptor which are deficient in an antigen to which the chimeric antigen receptor specifically binds will be "fratricide-resistant."

The term "genome-edited" as used herein means having a gene added, deleted, or modified to be non-functional. Thus, in certain embodiments, a "gene-edited CAR-T cell" is an CAR-T cell that has had a gene such as a CAR recognizing at least one antigen added; and/or has had a gene such as the gene(s) to the antigen(s) that are recognized by the CAR deleted; and/or has had a gene such as the TCR or a subunit thereof (e.g., the α or β chain) deleted or modified to be non-functional, or a subunit of the associated CD3 signal transduction complex, or a subunit thereof (e.g. the γ, δ, ε, or ζ chains) deleted or modified to be non-functional.

As used herein, "suicide gene" refers to a nucleic acid sequence introduced to a CAR-T cell by standard methods known in the art, that when activated result in the death of the CAR-T cell. If required suicide genes may facilitate the tracking and elimination, i.e., killing, of CAR-T cells in vivo. Facilitated killing of CAR-T cells by activating a suicide gene can be accomplished by standard methods known in the art. Suicide gene systems known in the art include, but are not limited to, include (a) herpes simplex virus (HSV)-tk which turns the nontoxic prodrug ganciclovir (GCV) into GCV-triphosphate, leading to cell death by halting DNA replication, (b) iCasp9 can bind to the small molecule AP1903 and result in dimerization, which activates the intrinsic apoptotic pathway, and (c) Targetable surface antigen expressed in the transduced T cells (e.g., CD20 and truncated EGFR), allowing eliminating the modified cells efficiently through complement/antibody-dependent cellular cytotoxicity (CDC/ADCC) after administration of the associated monoclonal antibody.

A "cancer cell", for example, is a malignant T cell, malignant B cell, or malignant plasma cell.

A "malignant B cell" is a B cell derived from a B-cell malignancy. B cell malignancies include, without limitation, (DLBCL), chronic lymphocytic leukemia (CLL) /small lymphocytic lymphoma (SLL), and B cell-precursor acute lymphoblastic leukemia (ALL).

A "malignant T cell" is a T cell derived from a T-cell malignancy.

The term "T-cell malignancy" refers to a broad, highly heterogeneous grouping of malignancies derived from T-cell precursors, mature T cells, or natural killer cells. Non-limiting examples of T-cell malignancies include T-cell acute lymphoblastic leukemia/lymphoma (T-ALL), , human T-cell leukemia virus type 1-positive (HTLV-1 +) adult T-cell leukemia/lymphoma (ATL), T-cell prolymphocytic leukemia (T-PLL), Adult T-cell lymphoma / leukemia (HTLV-1 associated), Aggressive NK-cell leukemia, Anaplastic large-cell lymphoma (ALCL), ALK positive, Anaplastic large-cell lymphoma (ALCL), ALK negative, Angioimmunoblastic T-cell lymphoma (AITL), Breast implant-associated anaplastic large-cell lymphoma, Chronic lymphoproliferative disorder of NK cells, Extra nodal NK / T-cell lymphoma, nasal type, Enteropathy-type T-cell lymphoma, Follicular T-cell lymphoma, Hepatosplenic T-cell lymphoma, Indolent T-cell lymphoproliferative disorder of the GI tract, Monomorphic epitheliotrophic intestinal T-cell lymphoma, Mycosis fungoides, Nodal peripheral T-cell lymphoma with TFH phenotype, Peripheral T-cell lymphoma (PTCL), NOS, Primary cutaneous γδ T-cell lymphoma, Primary cutaneous CD8+ aggressive epidermotropic cytotoxic T-cell lymphoma, Primary cutaneous acral CD8+ T-cell lymphoma, Primary cutaneous CD4+ small/medium T-cell lymphoproliferative disorders [Primary cutaneous anaplastic large-cell lymphoma (C-ALCL), lymphoid papulosis], Sezary syndrome, Subcutaneous, panniculitis-like T-cell lymphoma, Systemic EBV+ T-cell lymphoma of childhood, and T-cell large granular lymphocytic leukemia (LGL).

A "healthy donor," as used herein, is one who does not have a hematologic malignancy (e.g. a T-cell malignancy).

The term "therapeutically acceptable" refers to substances which are suitable for use in contact with the tissues of patients without undue toxicity, irritation, and allergic response, are commensurate with a reasonable benefit/risk ratio, and/or are effective for their intended use.

The term "therapeutically effective" is intended to qualify the amount of active ingredients used in the treatment of a disease or disorder or on the effecting of a clinical endpoint.

As used herein, a "secretable protein" is s protein secreted by a cell which has an effect on other cells. By way of example, secretable proteins include ctyokines, chemokines, and transcription factors.

The term "donor template" refers to the reference genomic material that the cell uses as a template to repair the a double-stranded break through the homology-directed repair (HDR) DNA repair pathway. The donor template contains the piece of DNA to be inserted into the genome (containing the gene to be expressed, CAR, or marker) with two homology arms flanking the site of the double-stranded break. In some embodiments, a donor template may be an adeno-associated virus, a single-stranded DNA, or a double-stranded DNA.

The term "exposing to," as used herein, in the context of bringing compositions of matter (such as antibodies) into intimate contact with other compositions of matter (such as cells), is intended to be synonymous with "incubated with," and no lengthier period of time in contact is intended by the use of one term instead of the other.

The term "patient" is generally synonymous with the term "subject" and includes all mammals including humans.

The invention is further illustrated by the following examples.

### EXAMPLES

### Example 1 - Method of Making and Testing a Genome-Edited CAR-T Cells by insertion of CAR into CD3e loci

The following steps may be taken to provide a genome-edited CAR-T cell in which the car is expressed from the gene edited loci (CAR-T) disclosed herein. This example describes the making of a CD7CART ΔCD7 ΔCD3ε cell. As those of skill in the art will recognize, certain of the steps may be conducted sequentially or out of the order listed below, though perhaps leading to different efficiency.

**Table 12. Guide RNA sequences for use in removing surface antigens on immune effector cells.**

| **Target gene** | **gRNA sequence** |
|---|---|
| CD7 | 5’_2′OMe(A(ps)U(ps)C(ps))ACGGAGGUCAAUGUCUAGUUUUAGAGCUAGA AAUAGCAAGUUAAAAUAAGGCUAGUCCGUUAUCAACUUGAAAAAGUG GCACCGAGUCGGUGC2′OMe(U(ps)U(ps)U(ps)U_3’ (SEQ ID NO:47) |
| CD7g10 | 5'_2'OMe(G(ps)U(ps)A(ps))GACAUUGACCUCCGUGAGUUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps)U_3' (SEQ ID NO:48) |
| CD7g4 | 5'_2'OMe(A(ps)U(ps)C(ps))ACGGAGGUCAAUGUCUAGUUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps)U _3'(SEQ ID NO:49) |
| TRACg | 5’_2'OMe(G(ps)A(ps)G(ps))AAUCAAAUCGGUGAAUGUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps)U_3’ (SEQ ID NO:50) |
| CS1 | 5'_2'OMe(G(ps)A(ps)C(ps))CAAUCUGACAUGCUGCAGUUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps)U_3' (SEQ ID NO:51) |
| CD2 | 5'_2'OMe(A(ps)C(ps)A(ps))GCUGACAGGCUCGACACGUUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps) U_3' (SEQ ID NO:52) |
| CD2g | 5'_2'OMe(G(ps)A(ps)G(ps))AAUCAAAAUCGGUGAAUGUUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps) U 3' (SEQ ID NO:53) |
| CD3εg | 5'_2'OMe(A(ps)G(ps)G(ps))GCAUGUCAAUAUUACUGGUUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps) U 3' (SEQ ID NO:54) |
| CD5 | 5’_2′OMe(C(ps)G(ps)U(ps))UCCAACUCGAAGUGCCAGUUUUAGAGCUAGA AAUAGCAAGUUAAAAUAAGGCUAGUCCGUUAUCAACUUGAAAAAGUG GCACCGAGUCGGUGC2′OMe(U(ps)U(ps)U(ps))U3’ (SEQ ID NO:55) |
| CD5g | 5'_2'OMe(C(ps)G(ps)U(ps))uCCAACUCGAAGUGCCAGUUUUAGAGCUAGA AAUAGCAAGUUAAAAUAAGGCUAGUCCGUUAUCAACUUGAAAAAGUG GCACCGAGUCGGUGC2'OMe(U(ps)U(ps)U(ps)U_3' (SEQ ID NO:56) |

| | |
|---|---|
| **RNA; (ps) indicate phosphorothioate. Underlined bases denote target sequence.** | |

### Step 1 - T Cell Activation (Day 0).

Purify T cells from leukapheresis chamber using Miltenyi human PanT isolation kit. Resuspend in media. Count cells. Determine number of human T cell activation CD3/CD28 beads required to obtain 3:1 bead:cell ratio. Wash beads 2x with T cell media. Dilute cells at 1.256 cells/mL in hXcyte media. Add human T cell activation CD3/CD28 beads. Aliquot 4 mL/well of 1.256 cell/mL solution into 6 well plate. Incubate cells at 37C.

### Step 2 - CRISPR (Day 2).

The target gene is genetically deleted and the CAR inserted into the gene edited loci. The DNA double strand break can be repaired using homolopgy directed rapair using a donor template to repair the break and insert the desired sequence into the editied loci. Target deletion may be accomplished by electroporating with Cas9 mRNA and gRNA against the target(s). The donor template may be, a DNA plasmid, or double stranded linear DNA containing homology to the DNA surrounding the double strand breaks electropoarted with the Cas9/gRNA. Additionally ,a viral vector such as AAV may be used as the source of the donor template. Other techniques, however, could be used to induce DNA double strand breaks. These include other genome editing techniques such as TALENs and mega-nucleases.

**Table 13. CRISPR Protocol**

| **Sample ID** | **gRNA#1** | **gRNA#2** | **Cas9** | **Nuecleofection Buffer P3** |
|---|---|---|---|---|
| **UCART7** | 20ug gCD7 | 20ug gCD3ε | 15ug Cas9 mRNA | 100ul |

*Protocol - Nucleofection using nucleofector 4D - 4x10⁶ cells per reaction.* Program EO-115 - 100ul transfection volume. The entire supplement needs to be added to the Nucleofector^{™} Solution P3. Prepare cell culture plates by filling appropriate number of wells with desired volume of recommended culture media (2ml in 6 well plate) and pre-incubate/equilibrate plates in a humidified 37 °C/5 % CO₂ incubator. Magnetically Remove beads (do this twice to ensure complete removal). Count cells and determine cell density. Centrifuge the required number of cells at 90xg for 10 minutes at room temperature. Remove supernatant completely. Resuspend in PBS (1ml) and transfer to a microcentrifuge tube and centrifuge the required number of cells at 90xg for 10 minutes at room temperature. Remove supernatant completely. Resuspend the cell pellet carefully in complete room temperature 4DNucleofector^{™} Solution P3 4x10⁶ per 100ul). Add 20ug of each gRNA **(gCD7** and **gCD3ε)** to each tube of 15ug cas9. Add 100ul of cells to each tube of Cas9/gRNA, gently mix and transfer everything into the Nucleocuvette^{™}. Gently tap to remove bubbles. Electroporate using program (Human T cell stim EO-115). After run completion, carefully remove the Nucleocuvette^{™} Vessel from the retainer using special. Resuspend cells with pre-warmed medium. Take up media from destination well, add to cuvette and gently pipetting up and down two to three times. Transfer to well. Repeat with media from same well. Incubate at 37°C.

### Step 3 - Transduction of T cells with AAV vector containing HDR repair construct.

Recombinant AAV6 donor vector is added to the cell culture 2-4hrs after electroporation with a MOI between 1e4 and 1e6.

### Step 4 - Assessment of CRISPR activity and Td efficiency (Day 10).

Take 5x10⁵ cells from each sample and analyze by flow cytometry. Wash samples with RB. Add 3ul of anti-CD34 PE antibody (This detects the CAR as our construct contains human truncated CD34). Add 5ul of CD3 APC and 2ul of CD2 BV421. Wash. Perform Flow cytometry. Cells should be CD3ε negative, CD7 negative. Harvest T cells **(Day 11).**

Purification of CAR-T cells. CD34+ (CAR+) and TCR negative cells can be purified in a single step using a positive selection of CD34+ cells on the Miltenyi Automacs. This enriches the CAR+ cells and removes and TCR+ cells (as CAR insertion disrupts TCR signaling)

### Step 5 - Assessment of CAR-T activity in vivo

Inject tumor in NSG mice (5e5 MOLT3 or HH: containing Luciferase) if performing in vivo imaging experiment. **(Day 7)**

Image tumor burden in mouse using bioluminescent imaging. Inject 2x10⁶ CD34+ CAR-T per mouse I.V. via tail vein or perform a 4hr chromium release assay against targets cell (MOLT3 or HH) **(Day 11).** Those of skill in the art will appreciate that some flexibility is possible in the time frames specified in Example 1.

### Example 2 - Method of Making a Genome-Edited Tandem tCAR- T Cells

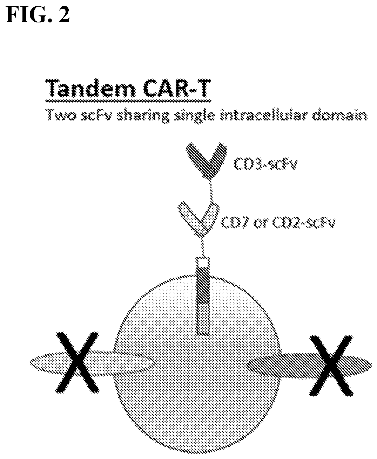

In a variation of the protocol in Example 1, a tandem CAR-T cell recognizing two antigens can be made. In Step 2, the two antigens can be deleted from the cell surface, or suppressed as described above, by electroporating with gRNA for each of the two targets and Cas9 mRNA. In Step 3, This CAR-T cell is then transduced with a CAR that recognizes two targets. The variations of a tandem CAR-T cell shown in the schematic in **FIG. 2****.** Additional examples of tCAR-T cells are shown in Table 6.

### Example 3 - Genome-Edited Dual CAR-T Cells or Genome-Edited Tandem CAR-T Cells

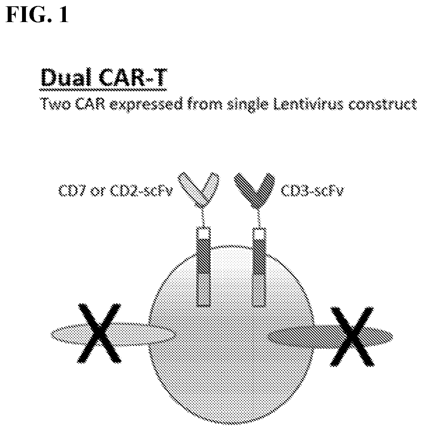

Several types of genome-edited dual or tandem CAR-T cells may be made using the methods above. **FIG. 1** and **FIG. 2** show the examples of tandem and dual CAR-T cells of the disclosure. The figures state the antigens to be targeted and does not indicate order of scFv expression in tandem CAR construct. Further examples are provided in Tables 6-11.

Additional examples of tandem and dual CAR-T cells are provided herein with deletion, without deletion, or suppression of one or more surface proteins that are target antigens of the CARs and expressed on CAR-T cells. In general, examples with deletion or suppression of more than one antigen will more likely have the benefit of greater fratricide-resistance for these CAR-T cells. It should be further noted that the order in which the scFvs are oriented in the tandem CARs are set forth in Tables 6-11 and is not limiting. For example, CD2*CD3ε encompasses a tCAR with the orientation CD2*CD3ε or the orientation CD3ε*CD2.

Additional examples of mono, tandem, and dual CAR-T cells targeting antigens expresses on multiple myeloma cells are provided herein, without deletion, with deletion, or suppression of one or more surface proteins that are target antigens of the CARs and expressed on CAR-T cells. In general, examples with deletion or suppression of more than one antigen will more likely have the benefit of greater fratricide-resistance for these CAR-T cells.

### Example 4 - Treatment of Patient(s) with Genome-Edited Dual or Tandem CAR-T Cells

Patients may be treated using cells made by the methods above, as shown in **FIG. 1** and **FIG. 2****.** For example, an expanded population of dual or tandem CAR-T cells may be infused into a patient

Dual or Tandem CAR-T cells target cancer cells without inducing alloreactivity. For example, CD2*CD3ε-dCARTΔCD2ΔCD3ε cells would target cancer cells (and other non-cancer cells) bearing the CD2 and CDε surface antigens.

### Example 6 - Genome-Edited Mono CAR-T cells

Examples of genome-edited mono CAR-T cells targeting antigens expresses on hematologic malignancies are provided below, without deletion, with deletion, or suppression of one or more surface proteins that are target antigens of the CARs and expressed on CAR-T cells. In general, examples with deletion or suppression of more than one antigen will more likely have the benefit of greater fratricide-resistance for these CAR-T cells.

The methods disclosed above can be varied appropriately by those skilled in the art to make and confirm activity of other mono, dual, and tandem CAR-T cells disclosed herein.

## Claims

1. A chimeric antigen receptor (CAR)-comprising T cell (CAR-T cell), which comprises one or more chimeric antigen receptors (CARs) targeting one or more antigens expressed on a malignant T cell, wherein a CAR specifically binds to CD7 antigen and comprises the amino acid sequence 
MALPVTALLLPLALLLHAARPDIQMTQTTSSLSASLGDRVTISCSASQGISNYLNWY
QQKPDGTVKLLIYYTSSLHSGVPSRFSGSGSGTDYSLTISNLEPEDIATYYCQQYSKLP
YTFGGGTKLEIKRGGGGSGGGGSGGGGSGGGGSEVQLVESGGGLVKPGGSLKLSCA
ASGLTFSSYAMSWVRQTPEKRLEWVASISSGGFTYYPDSVKGRFTISRDNARNILYL
QMSSLRSEDTAMYYCARDEVRGYLDVWGAGTTVTVSPRASTTTPAPRPPTPAPTIAS
QPLSLRPEACRPAAGGAVHTRGLDFACDFWVLVVVGGVLACYSLLVTVAFIIFWVK
RGRKKLLYIFKQPFMRPVQTTQEEDGCSCRFPEEEEGGCELRVKFSRSADAPAYKQG
QNQLYNELNLGRREEYDVLDKRRGRDPEMGGKPRRKNPQEGLYNELQKDKMAEA
YSEIGMKGERRRGKGHDGLYQGLSTATKDTYDALHMQALPPR,
 and wherein the CAR-T cell is deficient in a subunit of the T cell receptor complex and is deficient in CD7.

2. The CAR-T cell as recited in claim 1, wherein the subunit of the T cell receptor complex is chosen from TCRα, TCRβ, TCRδ, TCRγ, CD3ε, CD3γ, CD3δ, and CD3ζ.

3. The CAR-T cell as recited in claim 1 or 2, wherein the antigen expressed on a malignant T cell that is targeted by the subsequent of the one or more CARs is chosen from CD2, CD3, CD4, CD5, CD7, TCRA, and TCRβ.

4. A therapeutic composition comprising a population of CAR-T cells as recited in any of claims 1-3 and at least one therapeutically acceptable carrier and/or adjuvant.

5. The CAR-T cell as recited in any of claims 1-3 or the therapeutic composition as recited in claim 4 for use in a method of treatment of cancer in a patient in need thereof.

6. The CAR-T cell or therapeutic composition for use in the method as recited in claim 5, wherein the cancer is a hematologic malignancy.

7. The CAR-T cell or therapeutic composition for use in the method as recited in claim 6, wherein the hematologic malignancy is a T-cell malignancy.

8. The CAR-T cell or therapeutic composition for use in the method as recited in claim 7, wherein the T cell malignancy is T-cell acute lymphoblastic leukemia (T-ALL).

9. The CAR-T cell or therapeutic composition for use in the method as recited in claim 6, wherein the hematologic malignancy is acute myeloid leukemia (AML).

## Patentansprüche

1. Chimäre Antigen-Rezeptor(CAR-)umfassende T-Zelle (CAR-T-Zelle), die einen oder mehrere chimäre Antigen-Rezeptoren (CARs) umfasst, die ein oder mehrere Antigene targetieren, die auf einer malignen T-Zelle exprimiert werden, wobei ein CAR spezifisch an CD7-Antigen bindet und die Aminosäuresequenz 
MALPVTALLLPLALLLHAARPDIQMTQTTSSLSASLGDRVTISCSASQGISNYLNWY
QQKPDGTVKLLIYYTSSLHSGVPSRFSGSGSGTDYSLTISNLEPEDIATYYCQQYSKLP
YTFGGGTKLEIKRGGGGSGGGGSGGGGSGGGGSEVQLVESGGGLVKPGGSLKLSCA
ASGLTFSSYAMSWVRQTPEKRLEWVASISSGGFTYYPDSVKGRFTISRDNARNILYL
QMSSLRSEDTAMYYCARDEVRGYLDVWGAGTTVTVSPRASTTTPAPRPPTPAPTIAS
QPLSLRPEACRPAAGGAVHTRGLDFACDFWVLVVVGGVLACYSLLVTVAFIIFWVK
RGRKKLLYIFKQPFMRPVQTTQEEDGCSCRFPEEEEGGCELRVKFSRSADAPAYKQG
QNQLYNELNLGRREEYDVLDKRRGRDPEMGGKPRRKNPQEGLYNELQKDKMAEA
YSEIGMKGERRRGKGHDGLYQGLSTATKDTYDALHMQALPPR,
 umfasst, und wobei die CAR-T-Zelle in einer Untereinheit des T-Zell-Rezeptor-Komplexes defizient ist und in CD7 defizient ist.

2. CAR-T-Zelle nach Anspruch 1, wobei die Untereinheit des T-Zell-Rezeptor-Komplexes gewählt ist aus TCRα, TCRβ, TCRδ, TCRγ, CD3ε, CD3γ, CD3δ und CD3ζ.

3. CAR-T-Zelle nach Anspruch 1 oder 2, wobei das Antigen, das auf einer malignen T-Zelle exprimiert wird, die durch den nachfolgenden von dem einen oder den mehreren CARs targetiert wird, gewählt ist aus CD2, CD3, CD4, CD5, CD7, TCRA und TCRβ.

4. Therapeutische Zusammensetzung, umfassend eine Population von CAR-T-Zellen nach einem der Ansprüche 1-3 und zumindest einen therapeutisch annehmbaren Träger und/oder Hilfsstoff.

5. CAR-T-Zelle nach einem der Ansprüche 1-3 oder therapeutische Zusammensetzung nach Anspruch 4 zur Verwendung in einem Verfahren zur Behandlung von Krebs bei einem Patienten, der dessen bedarf.

6. CAR-T-Zelle oder therapeutische Zusammensetzung zur Verwendung in dem Verfahren nach Anspruch 5, wobei der Krebs eine hämatologische Malignität ist.

7. CAR-T-Zelle oder therapeutische Zusammensetzung zur Verwendung in dem Verfahren nach Anspruch 6, wobei die hämatologische Malignität eine T-Zell-Malignität ist.

8. CAR-T-Zelle oder therapeutische Zusammensetzung zur Verwendung in dem Verfahren nach Anspruch 7, wobei die T-Zell-Malignität akute lymphatische T-Zell-Leukämie (T-ALL) ist.

9. CAR-T-Zelle oder therapeutische Zusammensetzung zur Verwendung in dem Verfahren nach Anspruch 6, wobei die hämatologische Malignität akute myeloische Leukämie (AML) ist.

## Revendications

1. Lymphocyte T comprenant un récepteur d'antigène chimérique (CAR) (lymphocyte CAR-T), qui comprend un ou plusieurs récepteurs d'antigènes chimériques (CAR) ciblant un ou plusieurs antigènes exprimés sur un lymphocyte T malin, dans lequel un CAR se lie spécifiquement à l'antigène CD7 et comprend la séquence d'acides aminés 
MALPVTALLLPLALLLHAARPDIQMTQTTSSLSASLGDRVTISCSASQGISNYLNWY
QQKPDGTVKLLIYYTSSLHSGVPSRFSGSGSGTDYSLTISNLEPEDIATYYCQQYSKLP
YTFGGGTKLEIKRGGGGSGGGGSGGGGSGGGGSEVQLVESGGGLVKPGGSLKLSCA
ASGLTFSSYAMSWVRQTPEKRLEWVASISSGGFTYYPDSVKGRFTISRDNARNILYL
QMSSLRSEDTAMYYCARDEVRGYLDVWGAGTTVTVSPRASTTTPAPRPPTPAPTIAS
QPLSLRPEACRPAAGGAVHTRGLDFACDFWVLVVVGGVLACYSLLVTVAFIIFWVK
RGRKKLLYIFKQPFMRPVQTTQEEDGCSCRFPEEEEGGCELRVKFSRSADAPAYKQG
QNQLYNELNLGRREEYDVLDKRRGRDPEMGGKPRRKNPQEGLYNELQKDKMAEA
YSEIGMKGERRRGKGHDGLYQGLSTATKDTYDALHMQALPPR,
 et dans lequel le lymphocyte CAR-T est déficient en une sous-unité du complexe de récepteur de lymphocytes T et est déficient en CD7.

2. Lymphocyte CAR-T selon la revendication 1, dans lequel la sous-unité du complexe de récepteur de lymphocytes T est choisie parmi TCRα, TCRβ, TCRδ, TCRγ, CD3ε, CD3γ, CD3δ et CD3ζ.

3. Lymphocyte CAR-T selon l'une des revendications 1 ou 2, dans lequel l'antigène exprimé sur un lymphocyte T malin qui est ciblé par le ou les CAR suivants est choisi parmi CD2, CD3, CD4, CD5, CD7, TCRA et TCRβ.

4. Composition thérapeutique comprenant une population de lymphocytes CAR-T selon l'une quelconque des revendications 1 à 3 et au moins un support et/ou adjuvant acceptable thérapeutiquement.

5. Lymphocyte CAR-T selon l'une quelconque des revendications 1 à 3 ou composition thérapeutique selon la revendication 4, destiné(e) à être utilisé(e) dans un procédé de traitement d'un cancer chez un patient en ayant besoin.

6. Lymphocyte CAR-T ou composition thérapeutique destiné(e) à être utilisé(e) dans le procédé selon la revendication 5, ledit cancer étant une malignité hématologique.

7. Lymphocyte CAR-T ou composition thérapeutique destiné(e) à être utilisé(e) dans le procédé selon la revendication 6, ladite malignité hématologique étant une malignité à lymphocytes T.

8. Lymphocyte CAR-T ou composition thérapeutique destiné(e) à être utilisé(e) dans le procédé selon la revendication 7, ladite malignité à lymphocytes T étant une leucémie lymphoblastique aiguë à lymphocytes T (T-ALL).

9. Lymphocyte CAR-T ou composition thérapeutique destiné(e) à être utilisé(e) dans le procédé selon la revendication 6, ladite malignité hématologique étant une leucémie myéloïde aiguë (LMA).
